# EUROPÄISCHE PATENTANMELDUNG

(11) **EP 0 736 543 A2**
(43) Veröffentlichungstag der Anmeldung: **09.10.1996**
(21) Anmeldenummer: 96105373.3
(22) Anmeldetag: 03.04.1996
(51) Int. Cl.: C07K 16/00

(54) **Verfahren zur Herstellung von monoklonalen Antikörpern, die gegen proliferationsassoziierte Antigene des Zellkerns gerichtet sind**

(30) Priorität: 04.04.1995 DE 19512504
(71) Anmelder: BOEHRINGER MANNHEIM GMBH, D-68305 Mannheim-Waldhof (DE)
(72) Erfinder: Parwaresch, Reza, Prof. Dr., 24105 Kiel (DE)
(74) Vertreter: Weiss, Wolfgang, Dipl.-Chem. Dr.

(57) **Zusammenfassung**

Die Erfindung betrifft ein Verfahren zur Herstellung monoklonaler Antikörper, die mit proliferationsassoziierten Antigenen aus Zellkernen reagieren, sowie durch das Verfahren erhältliche Antikörper und deren Verwendung zum Nachweis proliferierender Zellen. Weiterhin betrifft die Erfindung Nucleotid- und Aminosäuresequenzen von proliferationsassoziierten Nucleinsäuren und Proteinen.

## Beschreibung

Die vorliegende Erfindung betrifft ein Verfahren zur Herstellung monoklonaler Antikörper, die mit proliferationsassoziierten Antigenen aus Zellkernen reagieren, sowie durch das Verfahren erhältliche Antikörper. Weiterhin betrifft die Erfindung Nucleotid- und Aminosäuresequenzen von proliferationsassoziierten Nucleinsäuren und Proteinen.

Zellbiologische Arbeiten im Rahmen der Tumorforschung konzentrierten sich viele Jahre auf die Tumorzellprodukte, wie etwa Katecholamine und Hormone. Mit der Einführung der Enzymcytochemie und der Verbesserung der Nachweismethoden rückten auch zunehmend membranassoziierte und cytoplasmatische Proteine, Lipide und Mucopolysaccharide in das Interessenfeld. Zur Identifizierung solcher Moleküle haben monoklonale Antikörper einen wertvollen Beitrag geleistet. Die mittlerweile erfolgte Verbesserung molekulargenetischer Methoden ermöglichte parallel dazu die Klonierung von Genen verschiedener Zellproteine. Dabei wurde deutlich, daß die Diversifikation des zellulären Phenotyps über eine differentielle Expression von Gengruppen u.a. durch eine zellspezifische DNA-Modifikation, wie die Methylierung, hervorgerufen und unterhalten wird.

Gegenwärtig konzentriert sich die Mehrzahl der analytischen Projekte auf die Aufklärung der komplizierten Mechanismen der Genregulation, die der Entwicklung eines der Funktion angepaßten zellulären Phenotyps dient. Hierunter fallen auch alle Vorgänge der Differenzierung, der Stoffwechselleistungen und der Zellproliferation. Nach neueren Untersuchungen wird auch der Zelltod durch interaktive Reaktionskaskaden reguliert.

Der Auffindung und Identifizierung von genregulierenden Proteinen stehen jedoch erhebliche Probleme im Wege. Insbesondere können die immunologischen Methoden der Proteinanalyse, die zu den wichtigsten zellbiologischen Untersuchungsmethoden gehören, nicht ohne weiteres eingesetzt werden. Bei einem großen Teil der im Zellkern vorhandenen DNA-bindenden Proteine handelt es sich nämlich um phylogenetisch hochkonservierte Strukturen mit großen Interspezieshomologien, die ihre Immunogenität bis zur vollen Toleranz einschränken. Daher ist die Herstellung von Antikörpern, die gegen derartige Proteine gerichtet sind, nur unter erheblichen Schwierigkeiten möglich.

Kreipe et al. (Am. J. Pathol. 142 (1993), 3-9) beschreiben ein proliferationsassoziiertes Antigen aus dem Zellkern mit einem Molekulargewicht von ca. 160 kD, das in Paraffin-eingebetteten Gewebeschnitten durch einen monoklonalen Antikörper mit der Bezeichnung Ki-S1 nachweisbar ist. Die Herstellung des Antikörpers Ki-S1 erfolgte unter Verwendung von lysierten U-937 Tumorzellen als Immunogen. Die Verwendung von lysierten Zellen bzw. Zellkernpräparationen als Immunogen hat jedoch den Nachteil, daß nur eine sehr geringe Anzahl von Antikörpern erzeugt wird, die spezifisch für proliferationsassoziierte Antigene sind.

Eine der vorliegenden Erfindung zugrundeliegende Aufgabe bestand somit darin, ein neues Verfahren zur Herstellung von Antikörpern gegen proliferationsassoziierte Antigene bereitzustellen, das die Nachteile des Standes der Technik mindestens teilweise vermeidet und das insbesondere eine höhere Effizienz als bereits bekannte Verfahren zeigt.

Diese Aufgabe wird gelöst durch ein Verfahren zur Herstellung monoklonaler Antikörper, die mit proliferationsassoziierten Antigenen aus Zellkernen reagieren, umfassend die Schritte:
(a) Herstellen einer Präparation von Zellkernen aus proliferierenden Zellen,
(b) Inkontaktbringen der Zellkernpräparation mit einem oder mehreren Präzipitationsantikörpern, die zur Bindung an Zellkernbestandteile von proliferierenden Zellen fähig sind, unter solchen Bedingungen, daß Immunkomplexe entstehen, die Präzipitationsantikörper und damit bindefähige Zellkernbestandteile enthalten,
(c) Isolieren der Immunkomplexe und gegebenenfalls Reinigen der isolierten Komplexe,
(d) Immunisieren von Versuchstieren mit den Komplexen aus Schritt (c) oder mit darin enthaltenen Zellkernbestandteilen,
(e) Herstellen von Hybridomzellen durch Fusion von Antikörperproduzierenden Zellen aus den immunisierten Versuchstieren mit immortalisierten Zellen,
(f) Selektionieren auf solche Hybridomzellen, die spezifisch zur Bindung an Zellkernbestandteile von proliferierenden Zellen fähige Antikörper produzieren, und
(g) Gewinnen von Antikörpern aus den selektionierten Hybridomzellen.

Durch das erfindungsgemäße Verfahren ist es überraschenderweise gelungen, eine größere Anzahl von monoklonalen Antikörpern herzustellen, die mit Antigenen aus Zellkernen proliferierender Zellen reagieren. Beispiele derartiger monoklonaler Antikörper sind die Antikörper S2, S3, S4 und Anti-p28/24, die von den bei der Deutschen Sammlung für Mikroorganismen und Zellkulturen GmbH, Mascheroder Weg 1b, 38124 Braunschweig, BRD (DSM) unter den Nummern DSM ACC 2200, DSM ACC 2205, DSM ACC 2206 und DSM ACC 2199 hinterlegten Hybridomzellinien produziert werden.

Der Antikörper S3 (DSM ACC 2205) ist vom Isotyp IgG1 erkennt ein Epitop auf dem Ki67-Antigen (p395/345). Ein weiterer gegen dieses Antigen gerichteter Antikörper Ki-S5 wurde bereits von Kreipe et al. (Am. J. Pathol. 142 (1993), 1689-1694) beschrieben. Das Ki67-Antigen ist ein Marker für die Proliferationsaktivität sowohl in normalen Zellen als auch in Tumorzellen. Der Antikörper S3 reagiert mit den Zellkernen humaner Zellen in den Zellzyklusphasen M, G2, S und G1. Er zeigt im wesentlichen keine Kreuzreaktivität mit anderen Antigenen.

Der Antikörper S4 (DSM ACC 2206) ist vom Isotyp IgG1 und erkennt ein Epitop auf der humanen Topoisomerase α II (p170). Der Antikörper S4 ist somit gegen das gleiche Antigen wie der bereits beschriebene Antikörper Ki-S1 (Kreipe et al., Am. J. Pathol. 142 (1993), 3-9) gerichtet. Auch die humane Topoisomerase II α ist ein Marker für die Proliferationsaktivität humaner Zellen. Der Antikörper S4 reagiert mit den Zellkernen humaner Zellen in den Zellzyklusphasen S, G2, M und G1. Er erkennt ein anderes Epitop auf der Topoisomerase als der Antikörper Ki-S1 und zeigt im Gegensatz zu diesem keine Kreuzreaktivität mit Epithelialzellen des Pankreas.

Der Antikörper S2 (DSM ACC 2200) ist vom Isotyp IgG1 und erkennt ein proliferationsassoziiertes Antigen aus dem Zellkern mit einem Molekulargewicht von ca. 100 kD (SDS-PAGE). Dieses Antigen wird nur von einem relativ geringen Anteil (ca. 20 - 40 %) der proliferierenden Zellen exprimiert, insbesondere von Zellen, die sich in den Zellzyklusphasen M, G2 und S befinden.

Der Antikörper-Anti-p28/p24 (DSM ACC 2199) ist vom Isotyp IgG1 und erkennt proliferationsassoziierte Antigene aus dem Zellkern mit Molekulargewichten von ca. 24 kD und 28 kD (SDS-PAGE). Diese Antigene können insbesondere in den Zellkernen von Spermatogonien und humanen Tumorzellen nachgewiesen werden.

Gemäß Schritt (a) des erfindungsgemäßen Verfahrens wird eine Präparation von Zellkernen aus proliferierenden Zellen hergestellt. Als Ausgangsmaterial verwendet man vorzugsweise permanente humane Zellinien oder humanes Tumorgewebe. Beispiele für geeignete permanente humane Zellinien sind U-937 (Sundström und Nilsson, Int. J. Cancer 17 (1976), 565-577), L-428 und L-580 (Diehl et al., J. Cancer Res. Clin. Oncol. 101 (1981), 111-124), NIH:OVCAR/3 (Hamilton et al., Cancer Res. 43 (1983), 5379-5389; ATCC H TB-61), He-La (Puck et al., J. Exp. Med. 103 (1956), 273-284; ATCC CCL-2) und SK-OV-3 (Fogh und Trempe in: Fogh I. (ed) Human tumor cells in vitro, pp. 155 - 195 (1975), Plenum Press, New York). U-937 ist eine histiocytäre Zellinie, die Eigenschaften unreifer Monozyten aufweist. L-428 und L-580 sind Zellinien, die aus dem Pleuraerguß eines Patienten mit M. Hodgkin gewonnen wurden. NIH: OVCAR/3 ist eine humane Ovarialkarzinom-Zellinie. Die He-La-Zellinie wurde von einem Plattenepithelkarzinom der Portio abgeleitet. Die Zellinie SK-OV-3 wurde von einem Adenokarzinom des Ovars etabliert.

Andererseits kann auch Tumorgewebe als Ausgangsmaterial zur Herstellung der Zellkernpräparation verwendet werden. Dieses Tumorgewebe kann während chirurgischer Operationen zu therapeutischen oder diagnostischen Zwecken gewonnen werden.

Zur Herstellung der Zellkernpräparation werden die cytoplasmatischen Bestandteile der Zellen vorzugsweise durch Behandlung mit einem nicht-ionischen Detergens, wie etwa Triton X-100 oder NP40, gegebenenfalls unter Zusatz von Proteaseinhibitoren, wie etwa Aprotinin, Pepstatin oder Leupeptin, mindestens teilweise lysiert. Die intakten Zellkerne können durch Zentrifugation gewonnen werden.

Gemäß Schritt (b) des erfindungsgemäßen Verfahrens wird die Zellkernpräparation mit einem oder mehreren Präzipitationsantikörpern in Kontakt gebracht, um die Bildung von Immunkomplexen hervorzurufen, welche an proliferationsassoziierten Kernantigenen angereichert sind. Dabei werden solche Präzipitationsantikörper verwendet, die zur Bindung an Zellkernbestandteile von proliferierenden Zellen fähig sind. Beispiele für geeignete Präzipitationsantikörper sind Antikörper, die zur Bindung mit der humanen Topoisomerase II, dem Ki67-Antigen, dem P100-Antigen oder/und den Antigenen p24 bzw. p28 fähig sind. Spezifische Beispiele sind die obengenannten Antikörper S2, S3, S4 und Anti-p24/p28, d. h. die durch das erfindungsgemäße Verfahren hergestellten Antikörper können in einem weiteren Verfahrenszyklus einzeln oder in Kombination als Präzipitationsantikörper zur Gewinnung neuer Antikörper verwendet werden.

Überraschenderweise wurde dabei festgestellt, daß man durch Verwendung eines Präzipitationsantikörpers mit einer vorgegebenen Spezifität Immunkomplexe erzeugen kann, die auch nicht mit dem Präzipitationsantikörper direkt reagierende Bestandteile enthalten. So wurde beispielsweise unter Verwendung des Anti-Ki67-Antikörpers S3 (DSM ACC 2205) als Präzipitationsantikörper ein Immunkomplex isoliert, bei dessen Verwendung als Immunogen der Antikörper S4 (DSM 2206) erhalten wurde, der gegen humane Topoisomerase IIα gerichtet ist. Bei Verwendung von S4 als Präzipitationsantikörper konnte der gegen das P100-Antigen gerichtete Antikörper S2 (DSM ACC 2200) erzeugt werden und schließlich konnte bei Verwendung von S2 als Präzipitationsantikörper der Antikörper Anti-p28/24 (DSM ACC 2199) erzeugt werden.

Um die Isolierung der Immunkomplexe aus Präzipitationsantikörpern und Zellkernbestandteilen zu erleichtern, wird vorzugsweise neben den Präzipitationsantikörpern eine damit bindefähige Festphase, z. B. eine partikuläre Festphase, zugegeben. Beispiele solcher partikulärer, mit dem Präzipitationsantikörper bindefähiger Festphasen sind Streptokokkenprotein A-beschichtete Sepharose in Kombination mit Anti-Maus-Ig (wenn der Präzipitationsantikörper ein Maus-Antikörper ist) oder mit Anti-Maus-Ig-beschichtete Agarose. Weiterhin kann man neben den Präzipitationsantikörpern noch ein Antiserum zugeben, das spezifisch an die Präzipitationsantikörper bindet. Ein derartiges Antiserum kann z. B. durch Immunisierung von Hühnern mit dem Präzipitationsantikörper und Gewinnung von Präzipitationsantikörper-bindenden Antiserumbestandteilen aus dem Eigelb der immunisierten Hühner gewonnen werden.

Die Isolierung der Immunkomplexe, die Präzipitationsantikörper und Zellkernbestandteile enthalten, erfolgt vorzugsweise durch Bindung der Präzipitationsantikörper an die Festphase und anschließende Trennung von Festphase und Überstand, z. B. durch Zentrifugation. Anschließend kann gegebenenfalls noch ein Reinigungsschritt zur Entfernung der Festphasenteilchen aus der Immunogenpräparation erfolgen. Dieser Schritt ist jedoch nicht obligatorisch.

Gemäß Schritt (d) des erfindungsgemäßen Verfahrens werden die Versuchstiere mit den Immunkomplexen oder mit darin enthaltenen Zellkernbestandteilen immunisiert. Diese Immunisierung erfolgt auf übliche Weise, gegebenenfalls unter Verwendung von Adjuvanzien. Als Versuchstiere werden vorzugsweise Nagetiere, insbesondere Mäuse, z. B. Balb/c-Mäuse verwendet.

Nach Abschluß der Immunisierung werden gemäß Schritt (e) des erfindungsgemäßen Verfahrens Hybridomzellen durch Fusion von Antikörper-produzierenden Zellen aus den immunisierten Versuchstieren mit geeigneten immortalisierten Zellen hergestellt. Vorzugsweise verwendet man Milzzellen aus den immunisierten Versuchstieren, die mit Myelomzellen, wie etwa der Plasmazytomzellinie X63-AG8.653, einem üblichen Fusionspartner zur Gewinnung monoklonaler Antikörper, fusioniert werden.

Gemäß Schritt (f) des erfindungsgemäßen Verfahrens selektioniert man auf solche Hybridomzellen, die spezifisch zur Bindung an Zellkernbestandteile von proliferierenden Zellen fähige Antikörper produzieren. Diese Selektion erfolgt vorzugsweise durch Inkontaktbringen der Hybridomüberstände mit Gewebe, das proliferierende Zellen enthält, z. B. mit Tumorgewebe oder/und menschlichen Tonsillen und anschließende Bestimmung der immunologischen Reaktivität. Hierzu werden vorzugsweise Kryostatschnitte von schockgefrorenen humanen Gewebestücken verwendet. Die Hybridomüberstände, die in den ersten Tests ein gewünschtes immunhistochemisches Muster ergeben, werden anschließend weiterhin auf ihre Spezifität getestet. Die Prüfungen der immunologischen Reaktivität und der Spezifizität der gewonnenen Antikörper erfolgen vorzugsweise mit Hilfe enzymatischer Anfärbungsmethoden, z. B. den Techniken der immunalkalischen Phosphatase (Cordell et al., J. Histochem. Cytochem. 32 (1984), 219-229) oder der Immunperoxidase.

Nach der Identifizierung von Hybridomzellen, welche einen Antikörper mit der gewünschten Spezifität erzeugen, erfolgt eine endgültige Monoklonalisierung, z. B. durch die Agarmethode von Cotton (Eur. J. Immunol. 3 (1973), 135-140).

Ein weiterer Gegenstand der vorliegenden Erfindung ist ein monoklonaler Antikörper, hergestellt durch das erfindungsgemäße Verfahren, oder ein Fragment eines solchen monoklonalen Antikörpers, z. B. ein Fab-, F(ab)₂- oder Fab'-Fragment. Derartige Antikörperfragmente können beispielsweise durch enzymatische Behandlung der monoklonalen Antikörper mit Proteasen, wie etwa Pepsin oder Papain, nach bekannten Methoden erhalten werden.

Erfindungsgemäße Antikörper bzw. Antikörperfragmente besitzen im wesentlichen keine Kreuzreaktivität mit cytoplasmatischem Material oder mit Zellkernbestandteilen aus nicht-proliferierenden Zellen. Im wesentlichen keine Kreuzreaktivität bedeutet, daß vorzugsweise ≦ 10 %, besonders bevorzugt ≦ 5 % und am meisten bevorzugt ≦ 2 % der nicht-proliferierenden Zellkerne markiert werden.

Durch das erfindungsgemäße Verfahren ist ein monoklonaler Antikörper erhältlich, der ein proliferationsassoziiertes Antigen aus dem Zellkern mit einem Molekulargewicht von ca. 100 kD (SDS-PAGE) erkennt. Dieser Antikörper reagiert vorzugsweise mit den Zellkernen humaner proliferierender Zellen, die sich in den Zellzyklusphasen S, G2 und M befinden. Er reagiert jedoch vorzugsweise im wesentlichen nicht mit humanen proliferierenden Zellen, die sich in der Zellzyklusphase G1 befinden. Ein Beispiel für einen Antikörper mit der oben genannten Spezifität ist der monoklonale Antikörper S2, produziert von der Hybridomzellinie DSM ACC 2200 oder ein monoklonaler Antikörper, der eine äquivalente Bindungsspezifität besitzt, d. h. ein Antikörper, der mit dem gleichen Antigen und vorzugsweise mit dem gleichen Epitop des Antigens reagiert.

Weiterhin ist durch das erfindungsgemäße Verfahren ein monoklonaler Antikörper erhältlich, der proliferationsassoziierte Antigene aus dem Zellkern mit Molekulargewichten von ca. 24 kD und 28 kD (SDS-PAGE) erkennt. Ein derartiger Antikörper reagiert vorzugsweise mit den Zellkernen humaner Tumorzellen, insbesondere mit Zellen von Keimdrüsentumoren, wie etwa Seminomen und Dysgerminomen. Der Antikörper reagiert vorzugsweise auch mit den Zellkernen von Spermatogonien. Er reagiert aber vorzugsweise im wesentlichen nicht mit den Zellkernen anderer humaner Zellen, wie etwa nicht-proliferierender Zellen oder somatischer proliferierender Zellen. Ein Beispiel für einen Antikörper mit derartiger Spezifität ist der monoklonale Antikörper Anti-p28/24, produziert von der Hybridomzellinie DSM ACC 2199 oder ein monoklonaler Antikörper, der eine äquivalente Bindungsspezifität besitzt, d. h. ein Antikörper, der mit den gleichen Antigenen und vorzugsweise mit den gleichen Epitopen der Antigene reagiert.

Auch die monoklonalen Antikörper S3 und S4, die von den Hybridomzellinien DSM ACC 2205 bzw. DSM ACC 2206 produziert werden, und monoklonale Antikörper, die das gleiche Epitop erkennen, sind durch das erfindungsgemäße Verfahren erhältlich.

Auch Konjugate der Antikörper oder Antikörperfragmente mit Markierungsgruppen oder pharmazeutisch aktiven Substanzen wie etwa Toxinen sind Gegenstand der vorliegenden Erfindung. Die kovalente Kopplung von Antikörpern mit derartigen Substanzen ist einem Fachmann geläufig (siehe z.B. Monoclonal Antibodies, J. H. Peters und H. Baumgarten (Eds.), Springer Verlag (1992), insbesondere Kapitel 9).

Die Antikörper, Antikörperfragmente und Konjugate können einzeln oder in Kombination auch in pharmazeutischen Zusammensetzungen für diagnostische oder therapeutische Zwecke eingesetzt werden. Neben der aktiven Komponente können die Zusammensetzungen gegebenenfalls übliche pharmazeutische Träger-, Verdünnungs- und Hilfsmittel enthalten.

Ein Gegenstand der vorliegenden Erfindung sind auch die mit erfindungsgemäßen Antikörpern reagierenden Antigene. Diese Antigene können durch ihre spezifische Reaktion mit den erfindungsgemäßen Antikörpern in reiner Form aus Zellkernextrakten isoliert werden, z. B. nach gelelektrophoretischer Auftrennung durch Ausschneiden der entsprechenden Bande aus dem Gel oder nach Leiten über eine Immunsorptionssäule mit immobilisierten erfindungsgemäßen Antikörpern und anschließende Elution.

Die Erfindung betrifft daher ein Antigen, das ein Molekulargewicht von ca. 100 kD (SDS-PAGE) aufweist und aufgrund einer Reaktion mit dem von der Hybridomzellinie DSM ACC 2200 produzierten monoklonalen Antikörper S2 aus Zellkernen humaner proliferierender Zellen, die sich in den Zellzyklusphasen S, G2 und M befinden, erhältlich ist.

Außerdem betrifft die Erfindung Antigene, die ein Molekulargewicht von ca. 24 kD oder 28 kD (SDS-PAGE) aufweisen und aufgrund einer Reaktion mit dem von der Hybridomzellinie DSM ACC 2199 produzierten monoklonalen Antikörper Anti-p28/24 aus Zellkernen humaner proliferierender Zellen, insbesondere Tumorzellen oder Spermatogonien erhältlich sind. Diese Antigene zeichnen sich weiterhin dadurch aus, daß sie an Nukleinsäuren mit der Sequenz (TTAGGG)ₙ oder Permutationen davon, z.B. (GGGTTA)ₙ, binden, wobei n eine natürliche Zahl ≧ 3 ist. Diese Sequenzen entsprechen den humanen Telomerase-Primersequenzen.

Vorzugsweise besitzen die mit dem Antikörper-Anti-p28/24 reagierenden Proteine eine oder mehrere der in den Sequenzprotokollen SEQ ID NO.1 bis 3 dargestellten Aminosäure-Teilsequenzen.

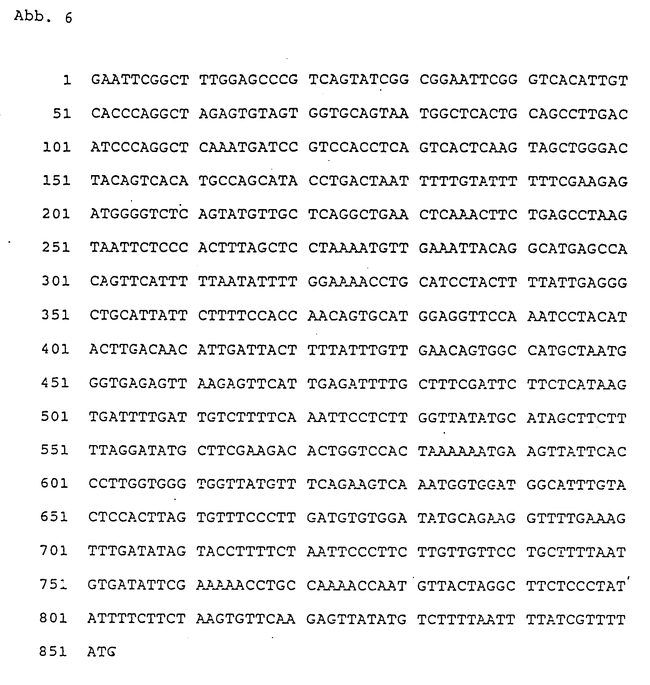

Noch ein weiterer Gegenstand der vorliegenden Erfindung sind Nukleinsäuren, welche (a) die in den Abbildungen 6, 7, 8 oder 9 dargestellte Nukleotidsequenzen, (b) spezifische Teilsequenzen mit einer Länge von mindestens 12, vorzugsweise von mindestens 15 Nukleotiden daraus oder (c) mit den Sequenzen aus (a) oder/und (b) unter stringenten Bedingungen hybridisierende Nukleotidsequenzen enthalten. Die in Abb. 6, 7, 8 und 9 bzw. in den Sequenzprotokollen SEQ ID NO. 4, 5, 6 und 7 gezeigten Nukleotidsequenzen codieren für proliferationsassoziierte Proteine oder Teilabschnitte davon und können als Sonden zur Messung der Genexpression, z. B. nach der Northern-Blot-Methode, eingesetzt werden, um die Proliferationsaktivität von Zellen, insbesondere von humanen Zellen zu bestimmen.

Unter stringenten Hybridisierungsbedingungen im Sinne der vorliegenden Erfindung versteht man, daß eine Hybridisierung auch nach Waschen bei 55°C, vorzugsweise bei 62°C und besonders bevorzugt bei 68°C, in einem wäßrigen Niedrigsalz-Puffer (z.B. 0,2 x SSC) noch auftritt (siehe auch Sambrook et al. (1989), Molecular Cloning. A Laboratory Manual, Cold Spring Harbor Laboratory Press). Vorzugsweise haben erfindungsgemäße Nukleinsäuren eine Homologie von mindestens 70%, besonders bevorzugt von mindestens 80% und am meisten bevorzugt von mindestens 90%, zu den in den Abbildungen 6, 7, 8 oder 9 dargestellten Nukleotidsequenzen.

Ein weiterer Gegenstand der vorliegenden Erfindung ist ein Vektor, der eine erfindungsgemäße Nukleinsäure enthält und eine Zelle, die mit mindestens einer Kopie einer erfindungsgemäßen Nukleinsäure oder eines erfindungsgemäßen Vektors transformiert ist.

Der Vektor kann ein beliebiger prokaryontischer oder eukaryontischer Vektor sein, auf dem sich die erfindungsgemäße DNA-Sequenz vorzugsweise unter Kontrolle eines Expressionssignals (Promotor, Operator, Enhancer, etc.) befindet. Beispiele für prokaryontische Vektoren sind chromosomale Vektoren, wie etwa Bakteriophagen (z. B. Bakteriophage λ) und extrachromosomale Vektoren, wie etwa Plasmide, wobei zirkuläre Plasmidvektoren besonders bevorzugt sind. Geeignete prokaryontische Vektoren sind bei Sambrook et al. (Molecular Cloning. A laboratory manual, Cold Spring Harbor Laboratory Press (1989), Kapitel 1 bis 4), beschrieben.

Andererseits kann der erfindungsgemäße Vektor auch ein eukaryontischer Vektor sein, z. B. ein Hefevektor ein für höhere Zellen geeigneter Vektor, z. B. ein Plasmidvektor, oder ein viraler Vektor. Derartige Vektoren sind dem Fachmann auf dem Gebiet der Molekularbiologie geläufig und beispielsweise bei Sambrook et al, Supra, Kapitel 16 beschrieben.

Die Zelle, die mit einer erfindungsgemäßen DNA-Sequenz oder einem erfindungsgemäßen Vektor transformiert ist, kann eine prokaryontische Zelle oder eine eukaryontische Zelle sein. Bevorzugte Beispiele für prokaryontische Zellen sind gram-negative prokaryontische Zellen, wie etwa Enterobakterienzellen und insbesondere E.coli-Zellen. Die Transformation prokaryontischer Zellen mit exogenen Nukleinsäuresequenzen ist einem Fachmann auf dem Gebiet der Molekularbiologie geläufig (vg. z. B. Sambrook et al., Supra, Kapitel 1 - 4).

Die erfindungsgemäße Zelle kann jedoch auch eine eukaryontische Zelle sein, wie etwa eine Pilzzelle (z. B. Hefe), eine tierische oder eine pflanzliche Zelle. Verfahren zur Transformation bzw.

Transfektion eukaryontischer Zellen mit exogenen Nukleinsäuresequenzen sind dem Fachman auf dem Gebiet der Molekularbiologie ebenfalls geläufig und brauchen hier nicht näher erläutert werden. (vgl. z. B. Sambrook et al., Kapitel 16).

Die erfindungsgemäßen Antikörper oder Antikörperfragmente können vorteilhaft als Marker zur Bestimmung der Proliferationsaktivität von Zellen verwendet werden. Die Antikörper S2, S3 und S4 und Antikörper äquivalenter Spezifität können beispielsweise als Prognosemarker bei Mammakarzinomen, bei der Beurteilung des endometrialen Zyklus, zur Abgrenzung benigner und maligner melanocytischer Hauttumoren, als Prognosemarker bei malignen Lymphomen oder/und zur Bestimmung der Antigen-stimulierten Proliferation von Lymphozyten verwendet werden.

So wurde bei Mammakarzinomen eine strenge Korrelation der Positivität von Tumorzellen für S-Antikörper (S2, S3, S4) mit der S-Phasenfraktion der untersuchten Zellen festgestellt. Eine Proliferationsaktivität von über 30 % der Tumorzellen korrelierte signifikant mit einer erhöhten Rezidivhäufigkeit und einer infausten Prognose. Die kumulative Überlebenswahrscheinlichkeit war in der Gruppe mit einer hohen S-Antikörperpositivität stark reduziert. In einem multivarianten Modell mit den Parametern S-Antikörper-Positivität der Tumorzellen, Lymphknotenmetastasen, Tumorgröße und Östrogen/Progesteron-Rezeptor-Expression wurde festgestellt, daß der Prozentsatz S-Antikörper-positiver Tumorzellen den zuverlässigsten unabhängigen Prognosemarker darstellt. In einer univarianten Analyse wurde eine Rezidivrate von 15 % bei Patientinnen beobachtet, die eine S-Antikörperpositivität von unter 30 % der Tumorzellen aufwiesen. Dagegen zeigten Patienten mit S-Antikörperpositivität von über 30 % der Tumorzellen eine Rezidivrate von 58 %.

Weiterhin können die S-Antikörper bei der Beurteilung des endometrialen Zyklus eingesetzt werden, da sie in den endometrialen Epithelzellen die proliferative Aktivität während der Proliferationsphase des Menstruationszyklus anzeigen. Zu Beginn des Menstruationszyklus wird eine Zunahme der Proliferationsaktivität im Endometrium beobachtet. Die höchsten Werte werden um die Ovulation und die geringsten Werte im Bereich des Sekretionsphase beobachtet. Die Proliferationsaktivität der stromalen Zellen, einschließlich der Macrophagen und Lymphocyten bleibt auch während der Sekretionsphase bestehen.

In Untersuchungen von 314 melanocytischen Tumoren hinsichtlich ihrer Reaktivität mit S-Antikörpern wurde festgestellt, daß gutartige Naevi eine Proliferationsaktivität unter 3 % aufwiesen. Werte über 3 % Proliferationsaktivität, gemessen am Prozentsatz S-positiver Tumorzellen wurden ausschließlich bei malignen Läsionen, nämlich Melanom in situ, invasivem Melanom unter 0,8 mm und invasivem Melanom über 0,8 mm sowie metastatischem malignem Melanom beobachtet. Die Antikörper S2, S3 und S4 können daher hervorragend zur Abgrenzung bzw. Unterscheidung benigner und maligner melanocytischer Hauttumoren eingesetzt werden.

Auch als Prognosemarker bei malignen Lymphomen sind die S-Antikörper geeignet. Es zeigte sich nämlich, daß sogenannte niedrig-maligne Lymphome wie chronische lymphatische Leukämie, lymphoplasmocytoides Immunocytom sowie zentroplastisch/zentrocytische Lymphome eine Positivität für S-Antikörper in unter 30 % der Tumorzellen aufweisen. Dagegen zeigen hochaggressive Lymphome, wie etwa das Burkitt-Lymphom, eine Proliferationsaktivität zwischen 60 und 95 %.

Weiterhin können die erfindungsgemäßen S-Antikörper zur Bestimmung der Antigen-stimulierten Proliferation von Lymphocyten eingesetzt werden, wie sie z.B. bei einer Immunstatusbestimmung durchgeführt wird. Überraschenderweise besitzen die erfindungsgemäßen Antikörper eine ausreichend hohe Sensitivität, um bei einem System mit einer derart geringen Proliferationsaktivität verwendet zu werden.

Der Antikörper Anti-p28/24 und äquivalent bindende Antikörper können als Prognosemarker bei Tumoren, insbesondere Keimdrüsentumoren wie etwa Seminomen und Dysgerminomen eingesetzt werden.

Besonders bevorzugt werden die erfindungsgemäßen Antikörper in einem immunologischen Proliferationsassay, insbesondere in einem ELISA-Proliferationsassay eingesetzt. Ein Gegenstand der vorliegenden Erfindung ist somit ein Verfahren zur Bestimmung der Proliferationsaktivität von Zellen, wobei man gegebenenfalls vorbehandelte Zellen mit mindestens einem erfindungsgemäßen Antikörper, Antikörperfragment oder Konjugat inkubiert und die Bindung des Antikörpers, des Antikörperfragments oder des Konjugats an die Zellen bestimmt. Die Vorbehandlung umfaßt vorzugsweise die Schritte des Fixierens oder/und Permeabilisierens der Zellen. Derartige Maßnahmen sind dem Fachmann geläufig und müssen daher nicht näher erläutert werden.

Die Zellen werden im allgemeinen aus dem Körper, z.B. aus Blut, Serum, Plasma oder anderen Körpermaterialien gewonnen und zum Test in ein geeignetes Medium, z.B. ein Kulturmedium oder einen physiologischen Puffer, gegeben. Der Test kann direkt nach Entnahme der Zellen oder erst nach Kultivierung der Zellen in vitro erfolgen. Das Medium kann gegebenenfalls mit proliferations- bzw. wachstumsbeeinflußenden Mediatoren versetzt sein.

Zur Bestimmung der Proliferationsaktivität von Zellen können direkt oder indirekt markierte Antikörper bzw. Antikörperfragmente eingesetzt werden. Direkt markierte Antikörper können durch kovalente Verknüpfung des Antikörpers mit einer geeigneten Markierungsgruppe erzeugt werden. Indirekt markierte Antikörper werden durch Bindung des Nachweisantikörpers an einen markierten Rezeptor, z. B. einen spezifischen Antikörper erzeugt. Vorzugsweise werden nicht-radioaktive Markierungsgruppen, insbesondere enzymatische Markierungsgruppen, wie etwa Peroxidase, alkalische Phosphatase oder Beta-Galactosidase, fluoreszenzchemische Markierungsgruppen oder Lumineszenzmarkierungsgruppen eingesetzt.

Weiterhin wird die Beschreibung durch die folgenden Beispiele in Verbindung mit den Abbildungen 1 bis 7 und den Sequenzprotokollen 1 bis 7 erläutert.

Es zeigen:
- Abb. 1:: das Ergebnis eines nicht-radioaktiven ELISA-Proliferations-Assay mit dem Antikörper S3,
- Abb. 2:: die Korrelation zwischen der im nicht-radioaktiven ELISA-Lymphozyten-Proliferations-Assay gemessenen Absorption und der durch immunhistochemische Untersuchungen bestimmten Anzahl proliferierender Zellen,
- Abb. 3:: die Korrelation des nicht-radioaktiven ELISA-Lymphozyten-Proliferations-Assay mit dem Einbau von 3H-Thymidin in die untersuchten Zellen,
- Abb. 4:: das Ergebnis eines ELISA-Proliferations-Assay in Abhängigkeit des Mischungsverhältnisses zwischen stimulierten und unstimulierten Zellen,
- Abb. 5:: eine Aminosäureteilsequenz eines mit dem Antikörper Anti-p24/p28 reagierenden Polypeptids,
- Abb. 6-9:: Nukleinsäuresequenzen, die für proliferationsassoziierte Polypeptide oder Teile davon kodieren,
- SEQ ID N0. 1: eine Aminosäureteilsequenz eines mit dem Antikörper Anti-p28/24 reagierenden Polypeptids,
- SEQ ID N0. 2: eine Aminosäureteilsequenz eines mit dem Antikörper Anti-p28/24 reagierenden Polypeptids,
- SEQ ID N0. 3: eine Aminosäureteilsequenz eines mit dem Antikörper Anti-p28/24 reagierenden Polypeptids (entsprechend Abb. 5),
- SEQ ID NO. 4: die vollständige Nukleotidsequenz des in Abb. 6 gezeigten Klons 21.1,
- SEQ ID NO. 5: die vollständige Nukleotidsequenz des in Abb. 7 gezeigten Klons 22.1,
- SEQ ID NO. 6: die vollständige Nukleotidsequenz des in Abb. 8a und 8b gezeigten Klons a 10 und
- SEQ ID NO. 7: die vollständige Nukleotidsequenz des in Abb. 9 gezeigten Klons 18.1.

### Beispiel 1

### Präparation von Zellkernextrakten aus Zellinien oder Tumorgewebe

Die unter Beispiel 1.1 - 1.3 beschriebenen Methoden können zur Anreicherung von Zellkernproteinen aus proliferierenden Zellen wie etwa Zellinien oder Tumorzellen eingesetzt werden.
1.1 Präparation der Kern-Proteine (Methode I)
5 x 10⁸-Zellen werden zweimal mit 50 ml 0,15 M Phosphatpuffer (PBS), pH 7,4 im Eisbad gewaschen, auf ein Endvolumen von 50 ml aufgefüllt und zentrifugiert (300 x g, 4°C, 10 Min.). Der Zellsuspension von 50 ml wird 0,1 % (V/V) des nichtionischen Detergens NP40 hinzugefügt. Nach 10 Min. wird durch eine mikroskopische Kontrolle sichergestellt, daß das Zytoplasma vollständig desintegriert ist. Die intakten Kerne werden zentrifugiert (300 x g, 4°C, 10 Min.), der Überstand abgehoben und das Pellet im Lysispuffer auf Eis inkubiert. Anschließend wird die Probe zentrifugiert (300 x g, 4°C, 10 Min.) und der Überstand jeweils weiterverwendet. Als Lysis-Puffer dient PBS versetzt mit 2 % (V/V) Triton-X100 und 1 mM Na₂EDTA. Außerdem werden die Protease-Inhibitoren 0,1 µM PMSF, 1 µM Aprotinin, 10 µM Pepstatin und 1 µM Leupeptin hinzugefügt.
1.2 Präparation der Kern-Proteine (Methode II)
5 x 10⁸-Zellen werden zweimal mit 50 ml 0,15 M PBS, pH 7,4 im Eisbad gewaschen und jeweils durch Zentrifugation (300 x g, 4°C, 10 Min.) sedimentiert. Das Pellet wird mit Lysis-Puffer überschichtet; als Lysis-Puffer dient hier erneut 0,15 M PBS, versetzt mit 2 % (V/V) TritonX100 und 1 mM Na₂EDTA. Zusätzlich werden Protease-Inhibitoren (1 µM PMSF, 1 µM Aprotinin, 10 µM Pepstatin und 1 µM Leupeptin) hinzugegeben. Nach 5 Min. wird die Probe zentrifugiert (10.000 x g, 5°C, 20 Sek.). Der Überstand enthält zytoplasmatische Teile und wird abgehoben. Das Pellet wird auf Eis mit einer 0,35 M NaCl unter Zusatz von 1 µM PMSF 30 Min. lang inkubiert. Die Zentrifugation der Probe (15.000 x g, 4°C, 10 Min.) trennt die DNA im Sediment und die nukleären Proteine im Überstand. Alle getrennten Fraktionen werden mindestens dreimal mit PBS gewaschen und zentrifugiert.
1.3 Extraktion nukleärer Proteine (Methode III)
5 x 10⁸ Zellen werden durch Zentrifugation (300 x g, 4°C, 5 Min.) pelletiert. Das Pellet wird in 1 ml Lysis-Puffer aufgefangen und erneut zentrifugiert (300 x g, 4°C, 5 Min.). Dieses Pellet wird in 750 µl Lysis-Puffer aufgefangen und mit 2 % (V/V) Triton X-100 versetzt. Innerhalb von 5 Min. der Inkubationszeit wird das Gemisch fünfmal auf Vortex vibriert und zentrifugiert (600 x g, 4°C, 5 Min.). Der Überstand wird als Zytoplasmalysat aufbewahrt. Das Pellet wird in 500 µl Extraktionspuffer (100 mM K-Phosphatpuffer pH 7,5; 1 M NaCl) aufgenommen und nach 5 Min. Inkubationszeit zentrifugiert (600 x g, 4°C, 5 Min.). Das Pellet wird gewogen und mit gleichem Volumen oder Gewicht Extraktionspuffer aufgefüllt. 15 µl einer 5 M NaCl-Lösung wird hinzugefügt und während der fünfminütigen Inkubationszeit fünfmal auf Vortex vibriert und zentrifugiert (30.000 x g, 4°C, 5 Min.).
Als Lysispuffer wird die im folgenden aufgeführte Zusammensetzung benutzt.

| Zusammensetzung des Lysispuffers: | |
|---|---|
| | Endkonzentration |
| Sucrose | 0.3 M |
| ECTA | 0.5 mM |
| KCl | 60 mM |
| NaCl | 15 mM |
| Spermin | 0.15 mM |
| Spermidin | 0.5 mM |
| HEPES | 15 mM |
| | pH 7,5 |
| β-Mercaptoethanol (14 mM) frisch vor Gebrauch hinzugeben Triton-X100 0,5% (V/V) frisch vor Gebrauch hinzugeben | |

### Beispiel 2

### Immunpräzipitation zur Anreicherung von proliferationsassoziierten Kernproteinen

Die unter Beispiel 2.1 - 2.2 beschriebenen Methoden können zur Immunpräzipitation von proliferationsassoziierten Zellkernproteinen aus den in Beispiel 1 hergestellten Kernextrakten verwendet werden.
2.1 Methode I der Immunpräzipitation zur Anreicherung von proliferationsassoziierten Kernproteinen, (SPA-Sepharose 4B-Methode)
   Die Kernproteinpräparationen gemäß Beispiel 1 können als Proteinquelle eingesetzt werden. 1,5 ml Streptokokkenprotein A(SPA)-Sepharose (Sigma) werden fünfmal mit 20 ml PBS bzw. TBS gewaschen und zentrifugiert (300 x g, 4°C, 10 Min.). 1,5 ml SPA-Sepharose werden mit 25 µl Kaninchen-Anti-Maus-Ig (Dianova, Z259) für 1 Std. auf Eis inkubiert. Anschließend wird die SPA-Sepharose fünfmal mit PBS gewaschen und im gleichen Volumen der Proteinpräparation suspendiert. 100 µl dieser Suspension werden mit 10 µl einer Lösung eines Präzipitationsantikörpers in einer Konzentration von 5 µg/µl versetzt und der Mischung 500 µl PBS hinzugefügt. Diese Probe wird mindestens eine, häufig vier und zum Teil acht Stunden bei 4°C inkubiert. Danach wird das Gemisch fünfmal mit 0,15 M PBS, pH 7,4 unter Zusatz von 0,05 % (W/V) Triton-X100 gewaschen, zentrifugiert (300 x g, 4°C, 10 Min.) und das Immunpräzipitat in 500 µl PBS resuspendiert. Diese Probe dient als Immunogen.
2.2 Methode II der Immunpräzipitation zur Anreicherung von proliferationsassoziierten Proteinen, (Agarose + Hühner-Antikörper-Methode)
   Es können die Kernproteinpräparate gemäß Beispiel 1 eingesetzt werden. Alternativ kann die Präzipitation auch mit 5 x 10⁸ zweimal gewaschenen Zellen in einer Suspension von 50 ml in 0,15 M PBS unter Zusatz von 50 µl NP40 (entsprechend einer 0,1 %igen Lösung) durchgeführt werden, danach muß NP40 5 - 10 Min. einwirken. Anschließend erfolgt eine mikroskopische Kontrolle der Zelldesintegration. Die Proben werden zentrifugiert (300 x g, 4°C, 10 Min.). Der Überstand wird entfernt. Das Pellet ist an Kernproteinen angereichert.
   Die Probe wird mit 5 ml einer Lösung des Präzipitationsantikörpers (1 µg/µl) versetzt, auf Eis 2 Std. geschüttelt und mit 1 ml einer Lösung eines aus Hühnereigelb gewonnenen, an den Präzipitationsantikörper bindenden Antiserums (1 µg/ml) versetzt und 30 Min. geschüttelt. Die Probe wird anschließend 8 Std. bei 4°C inkubiert.
   Zur Herstellung des Präzipitationsantikörper-bindenden Antiserums wurden 12 Wochen alte Hühner insgesamt achtmal in einwöchigem Abstand mit jeweils 500 µl einer Lösung des Präzipitationsantikörpers (10µg/ml) zusammen mit 250 µl komplettem Freund'schem Adjuvans immunisiert und das Antiserum (IgG-Fraktion) aus dem Eigelb von Eiern der immunisierten Hühner gewonnen.
   Dann wird die Probe mit einer konjugierten Agarose versetzt. Bei der konjugierten Agarose handelt es sich um ein Konjugat von Agarose mit Kaninchen-IgG, welches gegen Maus-IgG gerichtet ist (Sigma). 0,6 ml der konjugierten Agarosewerden in 1 ml PBS zweimal gewaschen, der Überstand verworfen und das Agarose-Konjugat dem obigen Gemisch (Kernproteinpräparat + Präzipitationsantikörper + Präzipitationsantikörperbindendes Antiserum) hinzugefügt.
   Die Probe wird 2 Std. bei Zimmertemperatur geschüttelt und 8 Std. bei 4°C inkubiert. Nach Zentrifugation (10.000 x g, 4°C, 10 Min.) wird das Pellet in 10 ml PBS über einem Vibrationsgerät aufgelöst und zur Immunisierung verwendet. Zur Kontrolle des Immunogens kann eine SDS-PAGE durchgeführt werden. Hierfür werden die Proben in 6 %iger (W/V) SDS in Elektrodenpuffer unter Zusatz von 1 µM Dithiothreitol (DTT) oder 5 % (W/V) Mercaptoethanol (MCE) bei 95°C für 5 Min. gekocht und zentrifugiert (10.000 x g, 4°C, 1 Min.), um die Sepharose- oder Agarose-Teilchen zu entfernen.
   Eine Entfernung der Sepharose- oder Agarose-Teilchen ist jedoch nicht obligat. Die angereicherten, DNA-assoziierten Regulatorproteine werden frisch zur Immunisierung von BALB/c-Mäusen herangezogen.

### Beispiel 3

Immunisierung von Versuchstieren und Herstellung der monoklonalen Antikörper S2, S3 und S4
3.1 Immunisierung
   Zur Immunisierung werden 6 Wochen alte weibliche BALB/c-Mäuse herangezogen. Diese erhalten einmal in der Woche eine intraperitoneale Injektion des Immunogens entsprechend 20 µg Eiweiß, gelöst in maximal 250 µl PBS unter Zusatz von 250 µl komplettem Freund'schen Adjuvans. Die Immunisierungsdauer beträgt etwa 50 Wochen.
3.2 Zellfusion zur Herstellung von monoklonalen Antikörpern (mAk)
   Eine Woche nach der letzten Immunisierung wird die Milz der Maus unter sterilen Bedingungen gewonnen und eine Zellfusion nach Angaben von Köhler und Milstein (Nature 256 (1975), 495-497) zur Gewinnung monoklonaler Antikörper durchgeführt. Als Fusionspartner dient die Plasmozytomzellinie X63-AG8.653. Die Fusion erfolgt über 90 Sek. mit 35 %igem (V/V) Polyethylenglykol (Serva) sowie 5 %igem (V/V) Dimethylsulfoxid (Merck) in RPMI 1640-Medium ohne Zusatz von fetalem Kälberserum unter leichtem Schwenken des Zentrifugenglases. Über die nächsten 10 Min. wird das Polyethylenglykol durch Zugabe von 10 ml und anschließend 20 ml RPMI ausverdünnt. Nach Passage durch das Selektivmedium werden Proben von Primärüberständen gewonnen und auf ihre immunologische Reaktivität mit Tumorgewebe sowie normalen menschlichen Tonsillen geprüft. Dabei werden 4 µm dicke Kryostatschnitte von schockgefrorenen humanen Gewebsstücken benutzt. Die endgültige Monoklonalisierung erfolgte durch die Agar-Methode (Cotton et al., Eur. J. Immunol. 3 (1973), 135-140).
3.3. Spezifitätstestung der erhaltenen monoklonalen Antikörper
   Die mAk, die in den ersten Testungen auf frischen Kryostatschnitten von Tumorgewebe sowie normalen Tonsillen ein gewünschtes immunhistochemisches Muster ergeben, werden anschließend auf Spezifität weiter getestet. Die Spezifitätsprüfung erfolgt mit Hilfe der immunhistochemischen Technik, wobei im wesentlichen die immunalkalische Phosphatase (L6) und die APAAP-Methoden nach Cordell et al. (J. Histochem. Cytochem. 32 (1984), 219-229) eingesetzt werden.
   Es wurde eine Immunogenpräparation mit einem Präzipitationsantikörper, der das Antigen Ki-67 (Gerdes et al., Int. J. Cancer 31 (1983), 13-20) erkennt, aber eine starke Kreuzreaktivität mit cytoplasmatischen Strukturen zeigt, hergestellt. Durch Immunisierung von BALB/c-Mäusen (Beispiel 3.1) und anschließende Zellfusion (Beispiel 3.2) wurde der monoklonale Antikörper S3 (DSM ACC 2205) erzeugt. Dieser Antikörper reagiert mit dem Ki-67 Antigen, besitzt aber keine cytoplasmatische Koreaktivität (Tabelle 1).
   In einem neuen Verfahrenszyklus wurde unter Verwendung von S3 als Präzipitationsantikörper der monoklonale Antikörper S4 (DSM ACC 2206) erzeugt. Dieser Antikörper reagiert mit humaner Topoisomerase II α und besitzt die in Tabelle 2 angegebene Spezifität.
   In einem weiteren neuen Verfahrenszyklus wurde unter Verwendung von S4 als Präzipitationsantikörper der monoklonale Antikörper S2 (DSM ACC 2200) erzeugt. Dieser Antikörper reagiert mit einem Antigen von ca. 100 kD (SDS-PAGE), das in Zellen der Zellzyklusphasen S, G2 und M exprimiert wird, und besitzt die in Tabelle 3 angegebene Spezifität.

**Tabelle 1**

| Immunhistochemische Spezifität des mAK S3 (DSM ACC 2205) | | |
|---|---|---|
| Gewebe | Proliferierende Zellen Reaktion im Kern | Nukleäre oder zytoplasmatische Koreaktivität |
| Haut | suprabasale Epidermis | - |
| ZNS/Nerven/Ganglien | - | - |
| Oropharynx | suprabasales Epithel, lymphatische Keimzentren | - |
| Herz u. Gefäße | Endothelien | - |
| Bronchopulmonales System | suprabasales Epithel, lymphatische Keimzentren | - |
| Gastrointestinaltrakt | Kryptengrund, lymphatische Keimzentren | - |
| Leber/Gallengänge | einzelne Hepatozyten und Gangepithelien | - |
| Pankreas | einzelne Epithelien | - |
| Nieren | einzelne Epithelien | - |
| Abl. Harnwege, Harnblase und Urethra | suprabasale Epithelien | - |
| Milz | lymphatische Keimzentren, Zentroblasten, Immunoblasten | - |
| Thymus | kortikale Thymozyten | - |
| Lymphknoten | Keimzentren, Zentroblasten, Immunoblasten | - |
| Endokrine Drüsen | einzelne Epithelien | - |
| Exokrine Drüsen | einzelne Epithelien | - |
| Hoden | Spermatogonien | - |
| Ovar | - | - |
| Uterus | Epithelien 3.-7. Zyklus-tag Stromazellen in der Sekretionsphase | - |

**Tabelle 2**

| Immunhistochemische Spezifität des mAK S4 (DSM 2206) | | |
|---|---|---|
| Gewebe | Proliferierende Zellen Reaktion im Kern | Nukleäre oder zytoplasmatische Koreaktivität¹ |
| Haut | suprabasale Epidermis | - |
| ZNS/Nerven/Ganglien | - | - |
| Oropharynx | suprabasales Epithel, lymphat. Keimzentren | - |
| Herz u. Gefäße | Endothelien | - |
| Bronchopulmonales System | suprabasales Epithel, lymphat. Keimzentren | - |
| Gastrointestinaltrakt | Kryptengrund, lymphat. Keimzentren | - |
| Leber/Gallengänge | einzelne Hepatozyten und Gangepithelien | - |
| Pankreas | einzelne Epithelien | - |
| Nieren | einzelne Epithelien | - |
| Abl. Harnwege, Harnblase und Urethra | suprabasale Epithelien | - |
| Milz | lymphat. Keimzentren, Zentroblasten, Immunoblasten | Sinus-Fasern |
| Thymus | kortikale Thymozyten | - |
| Lymphknoten | Keimzentren, Zentroblasten, Immunoblasten | - |
| Endokrine Drüsen | einzelne Epithelien | - |
| Exokrine Drüsen | einzelne Epithelien | - |
| Hoden | Spermatogonien | - |
| Ovar | - | - |
| Uterus | Epithelien 3.-7. Zyklus-tag Stromazellen in der Sekretionsphase | - |

| | | |
|---|---|---|
| ¹ = Koreaktion mit nicht-Zielantigen | | |

**Tabelle 3**

| Immunhistochemische Spezifität des mAK S2 (DSM ACC 2200) | | |
|---|---|---|
| Gewebe | Proliferierende Zellen Reaktion im Kern¹ | Nukleäre oder zytoplasmatische Koreaktivität |
| Haut | suprabasale Epidermis | - |
| ZNS/Nerven/Ganglien | - | - |
| Oropharynx | suprabasales Epithel, lymphatische Keimzentren | - |
| Herz u. Gefäße | Endothelien | - |
| Bronchopulmonales System | suprabasales Epithel, lymphatische Keimzentren | - |
| Gastrointestinaltrakt | Kryptengrund, lymphatische Keimzentren | - |
| Leber/Gallengänge | einzelne Hepatozyten und Gangepithelien | - |
| Pankreas | einzelne Epithelien | - |
| Nieren | einzelne Epithelien | - |
| Abl. Harnwege, Harnblase und Urethra | suprabasale Epithelien | - |
| Milz | lymphatische Keimzentren, Zentroblasten, Immunoblasten | - |
| Thymus | kortikale Thymozyten | - |
| Lymphknoten | Keimzentren, Zentroblasten, Immunoblasten | - |
| Endokrine Drüsen | einzelne Epithelien | - |
| Exokrine Drüsen | einzelne Epithelien | - |
| Hoden | Spermatogonien | - |
| Ovar | - | - |
| Uterus | Epithelien 3.-7. Zyklus-tag Stromazellen in der Sekretionsphase | - |

| | | |
|---|---|---|
| ¹ = Nur etwa 20-40 % der sicher proliferierenden Zellen werden durch S2 erkannt. | | |

### Beispiel 4

### ELISA-Proliferationsassay

Die in vitro Zellproliferation wird traditionell durch die Messung der Einbaurate von radioaktiv markierten DNA Vorläufern wie 3H-Thymidin gemessen. Diese Methode hat wesentliche Nachteile, wie den Bedarf an aufwendigen Geräten und den belastenden Umgang mit offenen Radioisotopen. Der Einsatz von Bromodeoxyuridin (BrdU)-Einbau anstelle von 3H-Thymidin und dessen Nachweis mit einem mAk (Magaud et al., J. Immunol. Methods 106 (1988), 95-100) wird verschiedentlich zur in vitro Zellproliferationsmessung benutzt. Die zeitraubende Vorbehandlung der Zellkulturen wird als Nachteil angesehen.

Monoklonale Antikörper gegen proliferationsassoziierte nukleäre Proteine wie Ki67/MiB1 oder anti-PCNA haben in der Tumorforschung gute Dienste geleistet, sie lassen sich jedoch für die Messung einer in-vitro-Zellproliferation nicht ohne weiteres einsetzen, da Ki67/MiB1 mit zytoplasmatischen Strukturen der Epithelzellen koreagieren (Gerdes et al. (1983), Supra) und das PCNA auch in nicht-proliferierenden Zellen oder in Zellen während der DNA-Reparatur exprimiert wird. Schließlich sind hochproliferierende Zellen bekannt, die kein PCNA exprimieren.

Der Antikörper S3 (DSM ACC 2205) erkennt ein Epitop des Ki67-Antigens P395/345. Die Reaktion ist streng und ohne Ausnahme an Zellkerne in den Zyklusphasen G1, S, G2 und M gebunden. Quiescente, nichtproliferierende G0 Zellen sind stets negativ. Ko- oder Kreuzreaktionen mit Nicht-Zielantigenen sind nicht bekannt. Nachfolgend werden Versuche beschrieben, die zeigen, daß es unter Einsatz von S3 möglich ist, ein Zell-ELISA System zur in-vitro-Erfassung der Proliferationsaktivität von Zellen zu entwickeln. Der hier durchgeführte ELISA-Proliferations-Assay (EPA) in 96-Well-Mikrotiterplatten wird parallel zu dem Standard 3H-Thymidin-Markierungsmethode vorgenommen, um die signifkante Korrelation beider Ergebnisse zu demonstrieren.

Frische Blutproben von freiwilligen Spendern beider Geschlechter wurden heparinisiert und über Dichtegradienten (Lymphoflot-Biotest, Dreieich) getrennt. Die Zellen wurden in RPMI 1640 (Gibco, Egenstein) suspendiert und mit 10 % (V/W) fetalem Kälberserum (FCS, Gibco), 5 % (W/V) L-Glutamin (Gibco) und 2 % (W/V) Gentamycin (Gibco) versetzt. Die Endkonzentration der Zellen betrug 5 x 10⁵/ml. 200 µl aliquote Proben wurden in 96 Well-Flachboden-Polystyrol-Mikrotiterplatten (Greiner, Frickenhausen) ausgesät. Die mitogene Stimulation wurde mit Phythämagglutinin (PHA, Biochrome, Berlin) in einer Endkonzentration von 5 µg/ml Kulturmedium durchgeführt. Die Kulturen wurden bei 37°C und einem CO₂- Anteil von 5 % der Atmosphäre für 4 Tage inkubiert. In Negativkontrollen wurde das PHA weggelassen.

Zu verschiedenen Zeitpunkten 1, 2 und 3 Tage nach der Stimulation wurden die Mikrotiterplatten zentrifugiert (300 x g, 4°C, 10 Min.) und die Überstände vollständig entfernt. Die Mikrotiterplatten wurden dann gründlich bei 37°C für 0,5 bis 2 Stunden getrocknet. 50 µl einer 0,1 %igen (V/V) Triton-X100-Lösung (Serva, Heidelberg) wurden hinzugefügt und nach 10 Min. entfernt. Anschließend wurden die Platten mit 0,1 M PBS, pH 7,4 unter Zusatz von 0,05 % (V/V) Tween 20 (Merck, Darmstadt) und 10 % (V/V) FCS (Waschmedium) gewaschen. Alle Waschvorgänge wurden mit einem automatischen Gerät (ELISA Processor II, Behring, Marburg) vorgenommen. 50 µl Überstand des den Antikörper S3 produzierenden Hybridoms (verdünnt 1:10 in 0,1 M PBS, pH 7,4) wurde in die Wells hineinpipettiert.

Die Mikrotiterplatte wurde für 30 Min. bei Zimmertemperatur inkubiert und dann dreimal automatisch gewaschen. 100 µl einer 4 %igen (V/V) Paraformaldehyd-Lösung in PBS wurde hinzugegeben und nach 10 Min. Inkubation durch dreimaliges Waschen mit Waschmedium gewaschen. 50 µl eines biotinylierten Kaninchen-anti-Maus Ig (E354, Dako, Hamburg) in einer Verdünnung von 1:1000 in 0,1 M PBS, pH 7,4 unter Zusatz von 5 % (V/V) hitzeinaktiviertem humanem Serum der Blutgruppe AB wurden hinzugegeben und 30 Min. bei Zimmertemperatur inkubiert. Erneut erfolgte eine dreimalige Waschung und Zusatz von 50 µl Streptavidin-Meerrettich-Peroxidase-Komplex (K377, Dako, Hamburg). Dieser Komplex wurde in einer Verdünnung von 1:100 in 0,1 M PBS bei pH 7,4 benutzt. Nach 30 Min. Inkubation bei Zimmertemperatur wurde die Mikrotiterplatte erneut dreimal gewaschen und mit 100 µl einer Lösung von 1-2-Phenylendiamin (OPD, S2000, Dako, Hamburg) versetzt. Dabei wurden 4 Tabletten mit je 2 mg OPD in 12 ml eines 0,1 M Zitronensäure-Phosphatpuffers, pH 5,0 gelöst.

Die Reaktion wurde nach 15 Min. durch Zusatz von 150 µl 1 M H₂SO₄ pro Well gestoppt. Die Absorption wurde durch einen automatischen ELISA-Processor (ELISA Processor II, Behring, Marburg) bei 492 nm gemessen. Alle Inkubationsschritte wurden bei Zimmertemperatur durchgeführt.

Kontrolluntersuchungen umfaßten immunhistochemische Kontrollen (Cordell et al., (1984), Supra) von Zytozentrifugenpräparaten, die am Ende der einzelnen Kulturperioden von Zellsuspensionen hergestellt wurden. Die Präparate wurden bei Zimmertemperatur für 24 Std. getrocknet, 5 Min. in Aceton fixiert und mit S3 gefärbt. Der Anteil S-Antikörper-positiver Zellen wurde unter 1.000 Zellen bestimmt.

Darüber hinaus wurden in Parallelkulturen 3 Std. vor Beendigung einer jeden Kulturperiode 0,2 mCi Methyl-3H-Thymidin (Amersham, Braunschweig) hinzugegeben. Für das Abernten der Zellen wurde ein Harvester (Scatron Combi, Scatron Lier, Norwegen) benutzt. Die Filter wurden in Szintillationsflüssigkeit durch ein LS-5000 td-Gerät (Beckmann, Hannover) gemessen.

Alle Versuche umfaßten mindestens 5 gesunde Spender. Alle Kulturen wurden in 6 aliquoten Proben vorgenommen. Negativkontrollen wurden in gleicher Weise durchgeführt, jeweils ohne PHA. Darüberhinaus wurde der Primärantikörper durch einen monoklonalen isotvpengleichen unspezifischen Antikörper ersetzt. Aussagen über signifikante Unterschiede wurden mit Hilfe des ungepaarten T-Testes geprüft. Als ein parameter freier Test wurde der Rangsummen-Test nach Mann-Whitney gewählt. Korrelationen wurden durch die Standard-Linearregression zum Ausdruck gebracht.

In den immunhistochemisch gefärbten Zytozentrifugenpräparaten aus den Kulturen 1 Tag nach der Stimulation betrug der Anteil positiver Zellen 0,1 %. Nach 48 Std. belief sich dieser Wert bereits auf 23,1 % ± 3,86 %. Nach 72 Std. wurde ein Mittelwert von 51 % ± 4,85 % festgestellt. Am Ende des 4. Tages nach der Stimulation betrug der Mittelwert positiver Zellen 72,5 % ± 3,2 %. Zu diesem Zeitpunkt waren beinahe alle Lymphozyten positiv. Bei den meisten negativen Zellen handelte es sich um Monozyten und Makrophagen. In den unstimulierten Kulturen blieb die Proliferationsrate bis zum 4. Tag unter 1 %. Bei den PHA-stimulierten Zellen wurde zwischen der Kulturzeit und der Anzahl positiver Zellen eine positive Korrelation mit einem Korrelationskoeffizienten von r = 0.98 bei p < 0,0001 festgestellt.

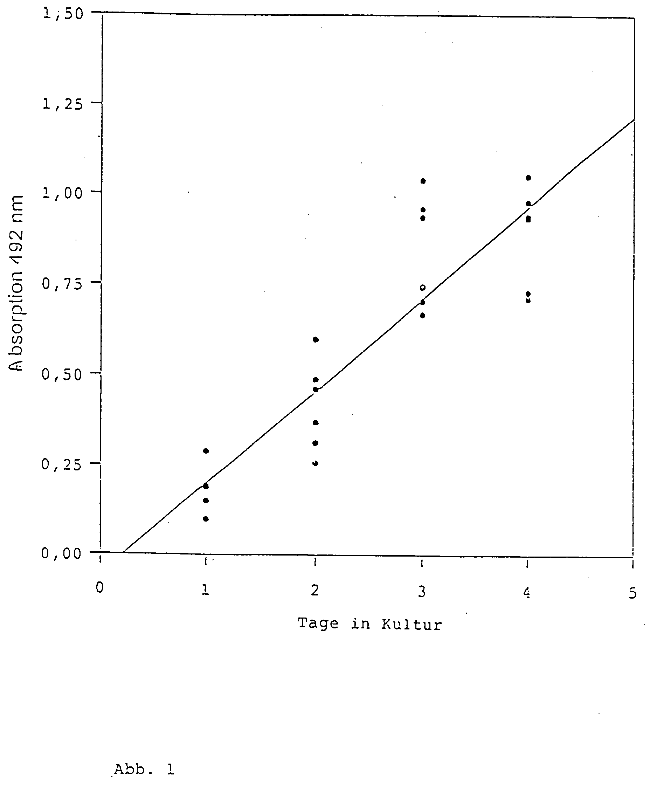

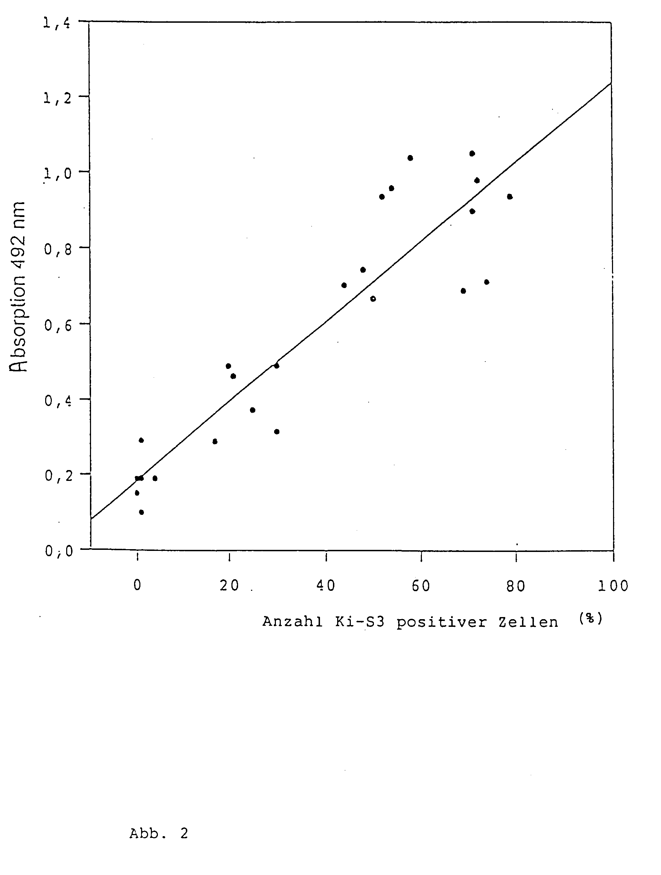

Parallel zu den immunhistochemischen Ergebnissen wurde auch im EPA eine tägliche Zunahme der Absorption während der Kulturzeit beobachtet. Diese Zunahme war linear und zeigte einen Regressionskoeffizienten von r = 0,98 bei p < 0,0001 (Abb. 1). Ein Vergleich zwischen dem Prozentsatz immunhistochemisch positiver Zellen und der ELISA-Absorption zeigte ebenfalls eine positive Korrelation mit r = 0,91 bei p < 0,0001 (Abb. 2).

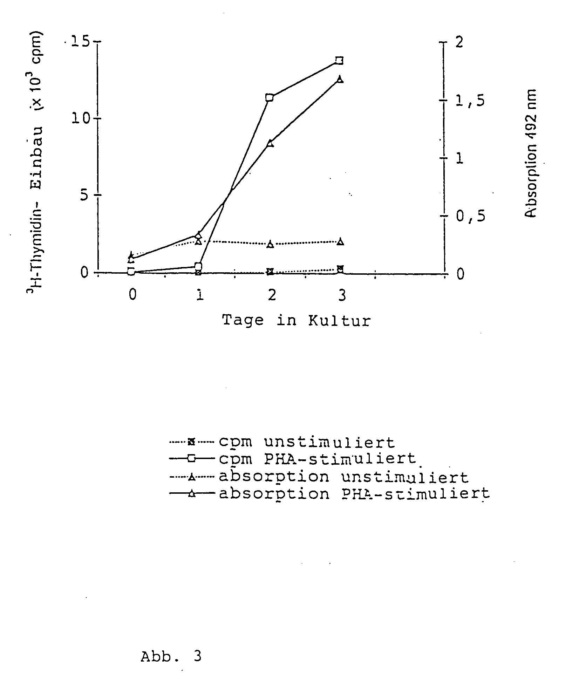

Aufschlußreiche Erkenntnisse brachte der Vergleich mit den Ergebnissen der 3H-Thymidin-Messungen (Abb. 3). Bereits nach 24 Std. wurde mit beiden Verfahren in den stimulierten Kulturen, nicht jedoch in den unstimulierten Kontrollkulturen ein leichter Anstieg beobachtet. 48 Stunden-Messungen zeigten einen deutlichen Anstieg mit beiden Messmethoden. Diese Tendenz blieb auch am 3. Tag erkennbar.

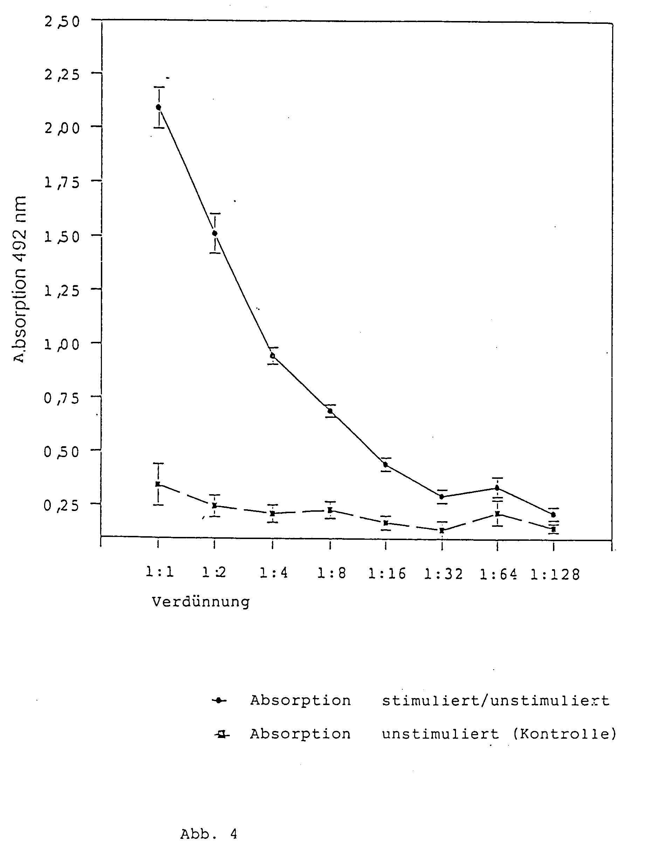

Um die Empfindlichkeit der Methode zu erfassen, wurden stimulierte Kulturen nach 3 Tagen mit unstimulierten Zellsuspensionen vermischt. Vor der Mischung wurde die Zellzahl jeweils auf 5 x 10⁵ Zellen pro ml eingestellt. Alle Messungen wurden in 6 Proben wiederholt. Die Werte wurden als Mittelwert zum Ausdruck gebracht. In Abb. 4 wurde die Abhängigkeit der Extinktion von dem Mischungsverhältnis mit unstimulierten Zellen dargestellt. Die kleinste Zahl positiver Zellen, die fü ein signifikantes Signal erforderlich sind, wird bei einer Verdünnung von 1:16 (p = 0,0022 Mann-Whitney-Test) erreicht. Mit anderen Worten, sind in jedem Well mindestens 4.500 positive Zellen erforderlich, damit ein signifikantes Signal über dem Testrauschen und einer Negativkontrolle verzeichnet wird. Diese Empfindlichkeitsgrenze läßt sich durch Verbesserung des Meßsignals unter Einsatz von Chemoluminiszenz oder Laser wesentlich herabsenken.

### Beispiel 5

### Herstellung und Charakterisierung des monoklonalen Antkörpers Anti p28/24 (DSM ACC 2199)

5.1 Herstellung von Anti-p28/24 (DSM ACC 2199)
In einem Verfahrenszyklus gemäß Beispiel 3 wurde unter Verwendung von S2 als Präzipitationsantikörper der monoklonale Antikörper Anti-p28/24 erzeugt.
Im Doppel-Immundiffusionstest gegen einen isotypen Kaninchen-anti-Maus-Antikörper erwies sich Anti-p28/24 als ein IgG1.
5.2 Immunhistochemische Reaktion mit Normalgewebe
Das Ergebnis einer immunhistochemischen Spezifitätsprüfung an Kryostatschnitten von frischen normalen Gewebsproben aus humanen Organen ist in Tabelle 4 gezeigt.
In den frischen, schockgefrorenen Gewebsproben aus der Haut, Oropharynx, verschiedenen Herzteilen, Arterien und Venen konnte keine Reaktivität mit Anti-p28/24 festgestellt werden. Gewebsproben aus dem Gastrointestinalbereich, einschließlich Pankreas, Leber und Gallenwegen ergaben ebenso eine negative Reaktion. Im Zentralnervensystem, in den Ganglien sowie in den peripheren Nerven wurde in den untersuchten Proben keine Reaktion beobachtet. In zwei Fällen dagegen wurde in dem weitgehend gesunden Nachbarbezirk eines malignen Hirntumors (Glioblastoma multiforme) in etwa 10 kapillären oder venolen Endothelien eine kräftige nukleäre Reaktion festgestellt. Alle anderen Gewebearten des normalen Gehirnes waren negativ. In den Lungen und dem Bronchialsystem, einschließlich der Trachea und dem Kehlkopf war das Gewebe stets negativ. Ebenso wurden in den Nieren und ableitenden Harnwegen, einschließlich der Harnblase und der Urethra keine positiven Zellen beobachtet.
Das Drüsensystem war stets negativ. Dies galt sowohl für die endokrinen wie für die exokrinen Drüsen.
In den lymphatischen Organen wie Thymus, Tonsille, Milz, Lymphknoten und Payer's Plaques und anderen Mukosa-assoziierten lymphatischen Geweben waren die Kerne aller untersuchten Zelltypen negativ. Lediglich in einigen wenigen Makrophagen und Plasmazellen wurde eine unregelmäßig schwache, zytoplasmatische Koreaktivität festgestellt. In allen diesen Zellen waren die Kerne frei von jeglicher Reaktion. Unter mehr als 500 Keimzentren der lymphatischen B-Follikel wurde in 2 Fällen in insgesamt 5 Zentroblasten eine im Kern lokalisierte Reaktion beobachtet.
Prostata, Samenblase, Nebenhoden, Zwischenzellen, Duktus deferens sowie Uterus, Portio, Vagina, Tuben, Fimbrien, Mesothelien des Ovars sowie das Interstitium des Ovars, einschließlich der Theka- und Granulosazellschicht sowie die Epithelien der reifen Follikel waren stets negativ. In einigen wenigen prämordialen Follikeln trat eine nukleäre schwache Reaktion auf. In den Hoden waren das Interstitium und die Tubulusepithelien negativ. Die Spermatogonien 1. und 2. Ordnung zeigten eine sehr intensive, auf die Kerne begrenzte Reaktion. Das Zytoplasma war stets negativ. Die reifen Spermatozoen waren ebenfalls negativ.

**Tabelle 4**

| Immunhistochemische Spezifität des monoklonalen Antikörpers Anti-p28/24 (DSM ACC 2199) | | |
|---|---|---|
| Gewebe | Reaktion | Koreaktivität |
| Haut | - | - |
| ZNS, Nerven, Ganglien | - | -¹ |
| Oropharynx | - | - |
| Herz, Venen, Arterien, | | |
| Kapillaren | - | - |
| Gastrointestinaltrakt | - | - |
| Lunge, Bronchien | - | - |
| Leber, Gallengänge | - | - |
| Pankreas | - | - |
| Nieren | - | - |
| Harnwege, Harnblase | - | - |
| Exokrine Drüsen | - | - |
| Endokrine Drüsen | - | - |
| Milz | - | - |
| Thymus | - | -² |
| Uterus | - | - |
| Ovar | unreife Oozyten+/- | - |
| Hoden | Spermatogonien+ | - |
| | Spermatozyten- | |

| | | |
|---|---|---|
| ¹ = In wenigen Endothelien der Hirnkapillaren wird selten eine nukleäre Koreaktivität festgestellt. | | |
| ² = 2 Lymphknoten unter 500 zeigten jeweils in 2 - 3 Zentroblasten eine nukleäre Positivität. Eine zytoplasmatische Koreaktivität wird in einigen wenigen Plasmazellen beobachtet. | | |

5.3 Immunhistochemische Reaktion von Anti-p28/24 mit Keimzelltumoren
Da im gesunden Gewebe Spermatogonien positiv waren, wurden zunächst die Keimzelltumore des Hoden untersucht, die bei Männern zwischen 15 und 50 Jahren die häufigsten malignen Tumore sind. In allen untersuchten Seminomen, Dysgerminomen und Gonadoblastomen wurde eine kräftige nukleär lokalisierte Reaktion festgestellt. Dottersacktumoren demonstrierten eine inhomogene Reaktion. Es wurden in dieser Gruppe 99 Fälle von Keimzelltumoren untersucht. Es handelte sich um 28 Seminome, 5 Seminommetastasen in den Lymphknoten, 17 Dottersacktumore und 3 embryonale Karzinome sowie 4 Dysgerminome, 2 Gonadoblastome und 2 Choriokarzinome. Darüberhinaus wurden 4 Fälle von reifen und 3 Fälle von unreifen Teratomen, wie auch 31 Fälle von malignen Keimzelltumoren mit mehr als einer histologischen Komponente, bestehend aus Dottersacktumor-, embryonalen Karzinom- und Choriokarzinomanteilen. Ferner wurden 7 Sertoli- und 3 Leydig-Zelltumoren und 1 Adenomatoidtumor immunhistochemisch analysiert.
Alle untersuchten Seminome, einschließlich Lymphknotenmetastasen dieser Tumoren zeigten eine kräftige nukleäre Immunreaktion. In wenigen Fällen war die Positivität schwach oder auf wenige Tumorzellen begrenzt. Dysgerminome waren vollständig oder teilweise positiv. Auch hier blieb die Reaktion auf die Tumorzellkerne begrenzt. Die Immunreaktivität von Anti-p28/24 in den Keimzelltumoren ist in der Tabelle 5 wiedergegeben.

**Tabelle 5**

| Reaktivität von Anti p28/24 mit Keimzelltumoren | | | | |
|---|---|---|---|---|
| **Monotypische Keimzelltumoren [Anzahl]** | +++ | ++ | + | - |
| Seminom [28] | 13 | 10 | 5 | 0 |
| Seminommetastasen [5] | 1 | 4 | 0 | 0 |
| Dysgerminom [4] | 1 | 2 | 1 | 0 |
| Gonadoblastom [2] | 2 | 0 | 0 | 0 |
| Dottersacktumor [17] | 1 | 5 | 2 | 9 |
| Choriokarzinom [2] | 0 | 1 | 0 | 1 |
| Embryonalkarzinom [3] | 0 | 0 | 0 | 3 |
| Teratom (reif) [4] | 0 | 0 | 0 | 4 |
| Teratom (unreif) [2] | 0 | 0 | 0 | 2 |

| **Gemischte Keimzelltumoren [31] (Komponenten)** | | | | |
|---|---|---|---|---|
| Seminom [9] | 4 | 4 | 1 | 0 |
| Dysgerminom [5] | 3 | 2 | 0 | 0 |
| Dottersacktumor [2] | 0 | 6 | 5 | 11 |
| Choriokarzinom [4] | 0 | 0 | 1 | 3 |
| Embryonalkarzinom [13] | 0 | 0 | 0 | 13 |
| Teratom (reif) [13] | 0 | 0 | 0 | 11 |
| Teratom (unreif) [3] | 0 | 0 | 0 | 2 |

### Beispiel 6

### Isolierung der Antigene p24 und p28

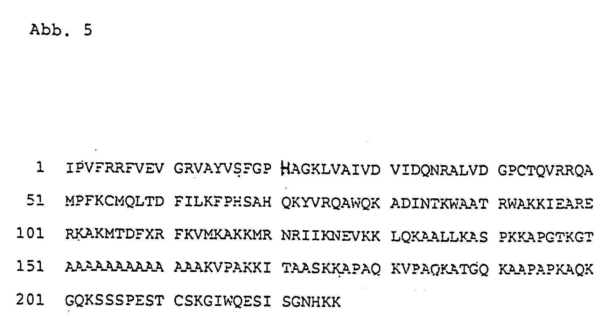

6.1 Immobilisierung von Anti-p28/24
   Der mAk Anti-p28/24 wurde über Affinitätssäulen gereinigt und kovalent an CNBr-aktivierter Sepharose 4B (Sigma) wie folgt gebunden:
   1 mg des gereinigten Antikörpers wurde an 0,5 mg CNBr-aktivierte Sepharose 4B gekoppelt. Ein "preclearing" erfolgte mit CNBr-aktivierter Sepharose 4B ohne Antikörper. 10⁸ p24/p28-positive Zellen (L-428, L-540 usw.) wurden in PBS bei 4°C suspendiert und mit 2 % (V/V) Triton X-100, 1 mM Na₂EDTA, 1 µM PMSF, pH 7,4 für 30 Min. inkubiert. Das Lysat wurde zentrifugiert (10.000 x g, 10 Min., 4°C). Der Überstand wurde zum "preclearing" mit Kaninchen-anti-Maus Ig gekoppelt an Protein-A-Sepharose C1 4B (Sigma) inkubiert. Das Kaninchen-anti-Maus Ig wurde von DAKO bezogen. Nach dem "preclearing" wurde das Anti-p28/24 gekoppelt an den Kaninchen-anti-Maus Ig- Protein A-Sepharose-Komplex dem Lysatüberstand hinzugefügt und bei 4°C 1 Stunde inkubiert. Nach dreimaligem Waschen wurde das Präzipitat 5 Min. gekocht in Probenpuffer (0,125 M Tris, 20 % (V/V) Glycerin, 6 % (W/V) SDS, pH 6,8).
   In parallelen Versuchen wurde ein Immunpräzipitat aus 5 x 10⁸ Zellen hergestellt. Nach fünfmaligem Waschen mit PBS bei 4°C wurde der Immunkomplex in einer Pufferlösung (Glycin/ HCl, pH 2,7 bei 4°C) für 30 Min. inkubiert. Der Komplex wurde dann zentrifugiert (20 Sek., 10.000 x g, 4°C) und der pH-Wert auf 7,4 eingestellt. Nach erneuter Zentrifugation (10.000 x g, 10 Min., 4°C) wurde die Probe zentrifugiert und in SDS-Probenpuffer aufgekocht.
   Nach SDS-PAGE wurden die Gele auf Polyvinylidenfluorid (PVDF)-Membranen (Millipor) geblottet und die Membranen mit Coomassie-Brilliant-Blue R250 (Biorad) gefärbt. Die zwei naheliegenden 24/28 kd-Banden wurden herausgeschnitten und in 100 µl Verdaupuffer (0,1 M Tris-HCl, pH 8,5, 1 µg Trypsin (Merck) über Nacht angedaut. Als Kontrolle diente ein benachbarter Membranbereich ohne Proteingehalt. Die proteolytischen Fragmente wurden durch "narrow-bore" HPLC (130 A, Applied Biosystems) mit Hilfe einer C4-Reverse-Phase-Säule (Vydac C4, 300 A Porengröße, 5 µ Partikelgröße, 2,1 x 100 ml) eluiert und mit einem linearen Gradienten (0-100 % B in 50 Min.; Lösungsmittel A: Wasser, 0,1% (W/V) Trifluoressigsäure (TFA); Lösungsmittel B: 70 % (V/V) Acetonitril, 0,09 % (W/V) TFA) mit einer Flußrate von 200 µl/Min. versetzt. Peptidhaltige Fraktionen wurden bei 214 nm manuell in silikonisierten Eppendorf-Röhrchen aufgefangen und sofort tiefgefroren. Die Aminosäuresequenzen wurden mit Hilfe der Standard-Edman-Degradation durch einen automatischen Sequenzer (473 A, Applied Biosystems) durchgeführt.
   Für die auf PVDF geblottete Probe wurden die in SEQ ID N0. 1 und SEQ ID N0. 2 dargestellten Aminosäureteilsequenzen ermittelt:
   SEQ ID N0. 1: PGXNAPVGGNVX
   Die Sequenzinformation von SEQ ID N0. 1 stammt nach vorläufigen Erkenntnissen von der 24 kD-Bande. Die Sequenz ist bisher unbekannt. Davon wurden die Positionen 5-11 herangezogen und 512 degenerierte Oligosonden synthetisiert (Oligonukleotidsonde I). Eine partielle Homologie scheint für die humane Ig-Leichtkette λ zu bestehen.
   SEQ ID N0. 2: VFRRFVEVGRVAYVSFGP
   Die Sequenzinformation von SEQ ID N0. 2 stammt nach vorläufigen Erkenntnissen von der 28 kD-Bande. Die 28 kD-Bande wurde in mehrfachen Elektrophoreseprozessen gereinigt und nach dem Blotten auf die PVDF-Membran einer direkten Aminosäuresequenzanalyse zugeführt. Eine N-terminale Sequenzierung ergab die in Abb. 5 und SEQ ID N0. 3 dargestellte Aminosäuresequenz.
   Die mit Anti-p28/24 reagierenden Antigene können auch über ihre Reaktion mit dem Telomer-Oligonukleotid (TTAGGG)ₙ oder Permutationen davon (n ≧ 3) isoliert werden. Hierzu können die Telomer-Oligonukleotide in biotinylierter Form an Streptavidin-beschichtete Magnetpartikel (Dynal A/S) gekoppelt werden, mit Zell-Lysaten gemäß Beispiel 1 inkubiert und anschließend von den nicht-bindenden Bestandteilen der Zell-Lysate abgetrennt werden.

### Beispiel 7

Isolierung von Nukleinsäuresequenzen, die für proliferationsassoziierte Antigene kodieren

Die Aminosäuresequenz NAPVGGNV wird zur Herstellung eines aus 512 degenerierten Oligonukleotidsonden bestehenden Gemisches (Oligonukleotidsonde I) herangezogen. Eine humane cDNA-Bibliothek im Bakteriophagen Lambda gt11 wurde nach Kultur auf Nitrocellulose- oder Nylonmembranen geblottet. Positive Klone, die durch Hybridisierung mit der Oligonukleotidsonde I identifizierbar waren, wurden getrennt hochgezüchtet und weiter gereinigt.

Anschließend erfolgte eine Umklonierung aus dem Bakteriophagenvektor in das Sequenzierungsplasmid Bluescript (Stratagene).

Auf diese Weise konnten mehrere positive Klone identifiziert werden, die für proliferationsassoziierte Polypeptide codierende Nukleinsäuren enthalten.

Der Klon 21.1 enthält eine cDNA-Sequenz von ca. 1300 bp. Ca. 900 bp vom 5'-Ende dieser Insertion wurden bereits sequenziert. Diese Sequenz ist in Abb. 6 dargestellt. Die komplette Sequenz dieses Klons ist in SEQ ID NO. 4 gezeigt.

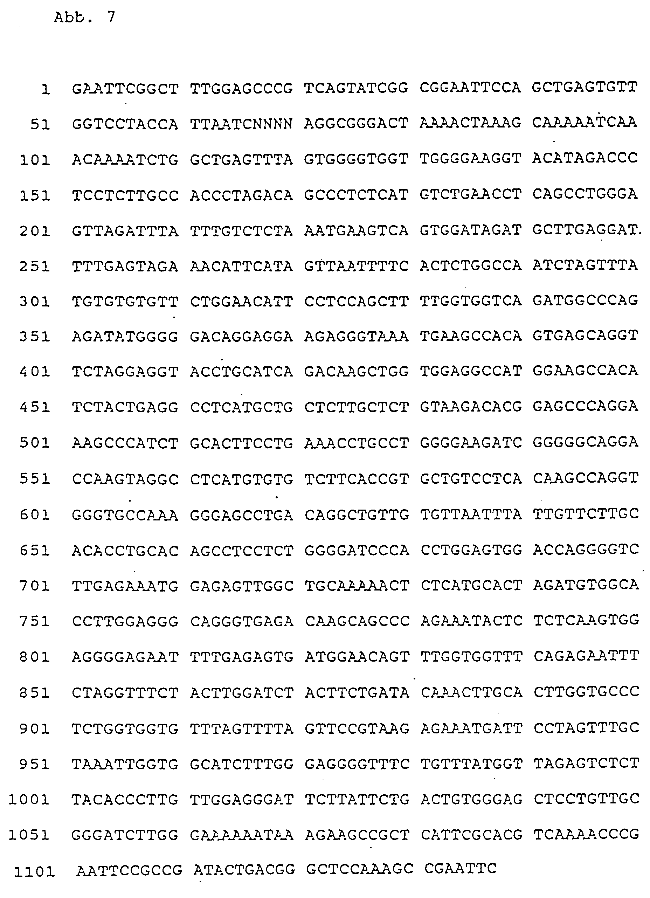

Der Klon 22.1 enthält eine cDNA-Sequenz von 1141 bp. Diese Insertion wurde bereits vollständig sequenziert und ist in Abb. 7 (SEQ ID NO. 5) dargestellt.

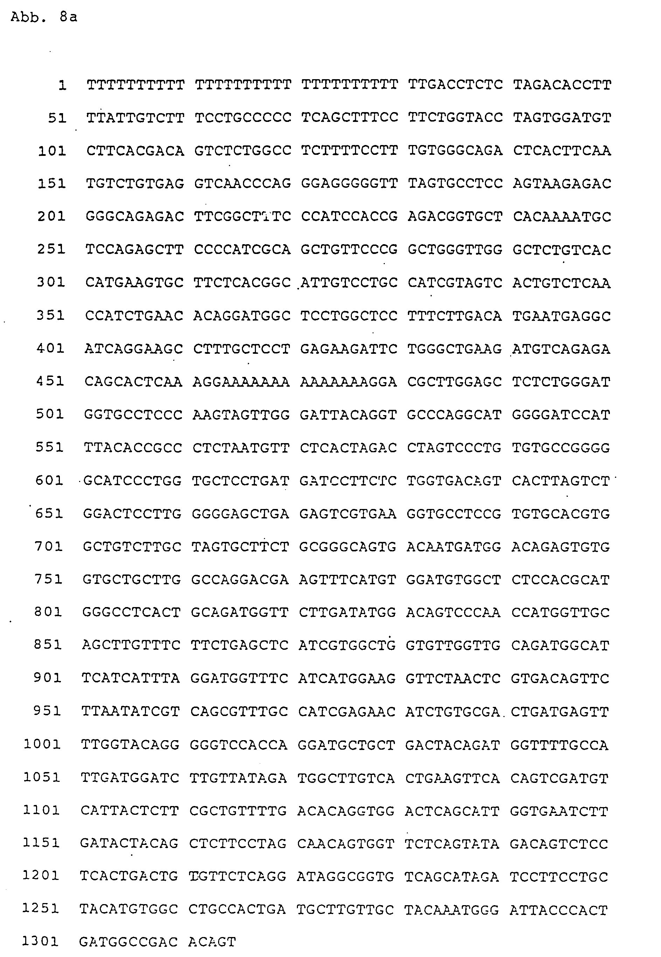

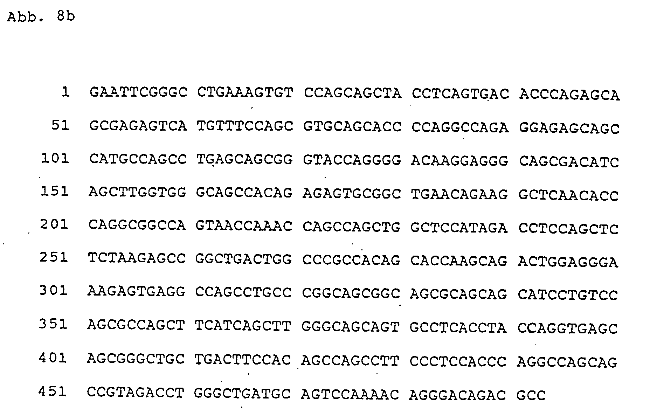

Der Klon a 10 enthält eine cDNA-Sequenz von ca. 2000 bp. Ca. 500 bp vom 3'-Ende und ca. 1300 bp vom 5'-Ende dieser Insertion wurden bereits sequenziert. Diese Sequenzen sind in Abb. 8a und 8b dargestellt. Die komplette Sequenz dieses Klons ist in SEQ ID NO. 6 gezeigt. Eine in situ-Hybridisierung der Sequenz ergab eine positive Reduktion mit Spermatogonien.

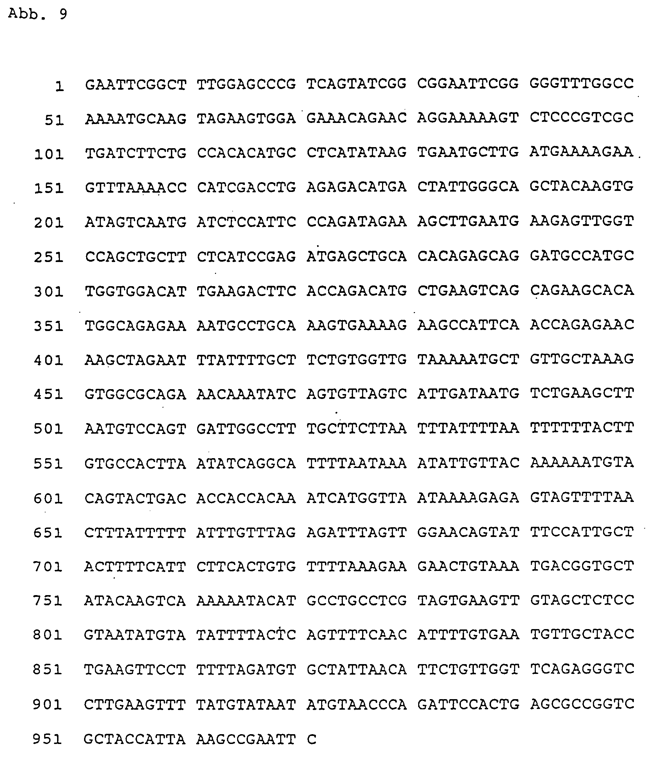

Der Klon 18.1 enthält eine cDNA-Sequenz von 971 bp. Diese Insertion wurde bereits vollständig sequenziert und ist in Abb. 9 (SEQ ID NO. 7) dargestellt.

## Patentansprüche

1. Verfahren zur Herstellung monoklonaler Antikörper, die mit proliferationsassoziierten Antigenen aus Zellkernen reagieren, umfassend die Schritte:
(a) Herstellen einer Präparation von Zellkernen aus proliferierenden Zellen,
(b) Inkontaktbringen der Zellkernpräparation mit einem oder mehreren Präzipitationsantikörpern, die zur Bindung an Zellkernbestandteile von proliferierenden Zellen fähig sind, unter solchen Bedingungen, daß Immunkomplexe entstehen, die Präzipitationsantikörper und damit bindefähige Zellkernbestandteile enthalten,
(c) Isolieren der Immunkomplexe und gegebenenfalls Reinigen der isolierten Komplexe,
(d) Immunisieren von Versuchstieren mit den Komplexen aus Schritt (c) oder mit darin enthaltenen Zellkernbestandteilen,
(e) Herstellen von Hybridomzellen durch Fusion von Antikörper-produzierenden Zellen aus den immunisierten Versuchstieren mit immortalisierten Zellen,
(f) Selektionieren auf solche Hybridomzellen, die zur Bindung an Zellkernbestandteile von proliferierenden Zellen fähige Antikörper produzieren, und
(g) Gewinnen von Antikörpern aus den selektionierten Hybridomzellen.

2. Verfahren nach Anspruch 1,
**dadurch gekennzeichnet,**
daß man humane Zellinien oder humanes Tumorgewebe als Ausgangsmaterial zur Herstellung der Zellkernpräparation verwendet.

3. Verfahren nach Anspruch 1 oder 2,
**dadurch gekennzeichnet,**
daß man zur Herstellung der Zellkernpräparation die cytoplasmatischen Bestandteile der Zellen durch Behandlung mit einem nicht-ionischen Detergens mindestens teilweise lysiert.

4. Verfahren nach einem der Ansprüche 1 bis 3,
**dadurch gekennzeichnet,**
daß man Präzipitationsantikörper verwendet, die zur Bindung mit der humanen Topoisomerase II, dem Ki67-Antigen, dem p100-Antigen oder/und den Antigenen p24 bzw. p28 fähig sind.

5. Verfahren nach einem der Ansprüche 1 bis 4,
**dadurch gekennzeichnet,**
daß man neben den Präzipitationsantikörpern eine damit bindefähige Festphase zugibt.

6. Verfahren nach Anspruch 5,
**dadurch gekennzeichnet,**
daß man eine partikuläre Festphase verwendet.

7. Verfahren nach einem der Ansprüche 1 bis 6,
**dadurch gekennzeichnet,**
daß man neben den Präzipitationsantikörpern ein Antiserum zugibt, das spezifisch an die Präzipitationsantikörper bindet.

8. Verfahren nach einem der Ansprüche 5 bis 7,
**dadurch gekennzeichnet,**
daß man die Präzipitationsantikörper und damit bindefähige Zellkernbestandteile enthaltenden Immunkomplexe durch Bindung der Präzipitationsantikörper an die Festphase und anschließende Abtrennung der Festphase isoliert.

9. Verfahren nach einem der Ansprüche 1 bis 8,
**dadurch gekennzeichnet,**
daß man die Hybridomzellen durch Fusion von Milzzellen aus den immunisierten Versuchstieren mit Myelomzellen herstellt.

10. Verfahren nach einem der Ansprüche 1 bis 9,
**dadurch gekennzeichnet,**
daß das Selektionieren auf Hybridomzellen, die spezifisch zur Bindung an Zellkernbestandteile von proliferierenden Zellen fähige Antikörper produzieren, durch Inkontaktbringen der Überstände aus Hybridomzellen mit Tumorgewebe oder/und menschlichen Tonsillen und anschließende Reaktivitätsbestimmung erfolgt.

11. Verfahren nach Anspruch 10,
**dadurch gekennzeichnet,**
daß die Bestimmung der Reaktivität von Hybridomüberständen an Kryostatschnitten unter Verwendung enzymatischer Anfärbungsmethoden erfolgt.

12. Monoklonaler Antikörper, hergestellt durch ein Verfahren nach einem der Ansprüche 1 bis 11, der im wesentlichen keine Kreuzreaktivität mit cytoplasmatischem Material oder mit Zellkernbestandteilen aus nicht-proliferierenden Zellen besitzt.

13. Monoklonaler Antikörper, der ein proliferationsassoziiertes Antigen aus dem Zellkern mit einem Molekulargewicht von ca. 100 kD (SDS-PAGE) erkennt und im wesentlichen keine Kreuzreaktivität mit cytoplasmatischem Material oder mit Zellkernbestandteilen aus nicht-proliferierenden Zellen besitzt.

14. Antikörper nach Anspruch 13,
**dadurch gekennzeichnet,**
daß er mit Zellkernen humaner proliferierender Zellen reagiert, die sich in den Zellzyklusphasen S, G2 und M befinden.

15. Antikörper nach Anspruch 13 oder 14,
**dadurch gekennzeichnet,**
daß er im wesentlichen nicht mit humanen proliferierenden Zellen reagiert, die sich in der Zellzyklusphase G1 befinden.

16. Monoklonaler Antikörper S2, produziert von der Hybridomzellinie DSM ACC 2200, oder ein monoklonaler Antikörper, der eine äquivalente Bindungsspezifität besitzt.

17. Monoklonaler Antikörper, der proliferationsassoziierte Antigene aus dem Zellkern mit Molekulargewichten von ca. 24 kD und 28 kD (SDS-PAGE) erkennt und im wesentlichen keine Kreuzreaktivität mit cytoplasmatischem Material oder mit Zellkernbestandteilen aus nicht-proliferierenden Zellen besitzt.

18. Antikörper nach Anspruch 17,
**dadurch gekennzeichnet,**
daß er mit den Zellkernen humaner Tumorzellen reagiert.

19. Antikörper nach Anspruch 18,
**dadurch gekennzeichnet,**
daß er mit Seminomen und Dysgerminomen reagiert.

20. Antikörper nach einem der Ansprüche 17 bis 19,
**dadurch gekennzeichnet,**
daß er mit den Zellkernen von Spermatogonien reagiert, aber im wesentlichen nicht mit den Zellkernen anderer normaler humaner Zellen reagiert.

21. Monoklonaler Antikörper Anti p28/24, produziert von der Hybridomzellinie DSM ACC 2199, oder ein monoklonaler Antikörper, der eine äquivalente Bindungsspezifität besitzt.

22. Monoklonaler Antikörper S3, produziert von der Hybridomzellinie ACC 2205, oder ein monoklonaler Antikörper der das gleiche Epitop erkennt.

23. Monoklonaler Antikörper S4, produziert von der Hybridomzellinie DSM ACC 2206, oder ein monoklonaler Antikörper, der das gleiche Epitop erkennt.

24. Fragment eines monoklonalen Antikörpers nach einem der Ansprüche 12 bis 23.

25. Konjugat eines monoklonalen Antikörpers nach einem der Ansprüche 12-23 oder eines Antikörperfragments nach Anspruch 24 mit einer Markierungsgruppe oder einer pharmazeutisch aktiven Substanz.

26. Pharmazeutische Zusammensetzung,
**dadurch gekennzeichnet,**
daß sie als aktive Komponente einen oder mehrere monoklonale Antikörper nach einem der Ansprüche 12-23, Antikörperfragmente nach Anspruch 24 oder Konjugate nach Anspruch 25, gegebenenfalls zusammen mit pharmazeutisch üblichen Träger-, Verdünnungs- und Hilfsmitteln enthält.

27. Antigen, das ein Molekulargewicht von ca. 100 kD (SDS-PAGE) aufweist und aufgrund einer Reaktion mit dem von der Hybridomzellinie DSM ACC 2200 produzierten monoklonalen Antikörper S2 aus Zellkernen humaner proliferierender Zellen, die sich in den Zellzyklusphasen S, G2 und M befinden, erhältlich ist.

28. Antigene, die ein Molekulargewicht von ca. 24 kD oder 28 kD (SDS-PAGE) aufweisen und aufgrund einer Reaktion mit dem von der Hybridomzellinie DSM ACC 2199 produzierten monoklonalen Antikörper Anti-p28/24 aus Zellkernen humaner proliferierender Zellen erhältlich sind.

29. Antigene nach Anspruch 28,
**dadurch gekennzeichnet,**
daß sie an Nukleinsäuren mit der Sequenz (TTAGGG)ₙ oder Permutationen davon binden, wobei n eine natürliche Zahl ≧ 3 ist.

30. Antigene nach Anspruch 28 oder 29,
**dadurch gekennzeichnet,**
daß sie eine oder mehrere der in den Sequenzprotokollen SEQ ID NO. 1 bis 3 dargestellten Aminosäuresequenzen enthalten.

31. Nukleinsäure,
**dadurch gekennzeichnet,**
daß sie (a) die in Abb. 6, Abb. 7, Abb. 8 oder Abb. 9 dargestellte Nukleotidsequenz, (b) eine Teilsequenz davon mit einer Länge von mindestens 12 Nukleotiden oder (c) eine mit einer Sequenz aus (a) oder/und (b) unter stringenten Bedingungen hybridisierende Nukleotidsequenz enthält.

32. Verwendung einer Nukleinsäure nach Anspruch 31 zur Messung der Proliferationsaktivität humaner Zellen.

33. Vektor,
**dadurch gekennzeichnet,**
daß er mindestens eine Kopie einer Nukleinsäure nach Anspruch 32 enthält.

34. Zelle,
**dadurch gekennzeichnet,**
daß sie mit mindestens einer Kopie einer Nukleinsäure nach Anspruch 31 oder eines Vektors nach Anspruch 33 transformiert ist.

35. Verwendung eines Antikörpers nach einem der Ansprüche 12 bis 23, eines Antikörperfragments nach Anspruch 24 oder eines Konjugats nach Anspruch 25 als Marker zur Bestimmung der Proliferationsaktivität von Zellen.

36. Verwendung nach Anspruch 35 als Prognosemarker bei Mammakarzinomen.

37. Verwendung nach Anspruch 35 bei der Beurteilung des endometrialen Zyklus.

38. Verwendung nach Anspruch 35 zur Abgrenzung benigner und maligner melanocytischer Hauttumoren.

39. Verwendung nach Anspruch 35 als Prognosemarker bei malignen Lymphomen.

40. Verwendung nach Anspruch 35 zur Bestimmung der Antigenstimulierten Proliferation von Lymphozyten.

41. Verwendung nach einem der Ansprüche 35 bis 40 in einem immunologischen Proliferationsassay, insbesondere einem ELISA.

42. Verfahren zur Bestimmung der Proliferationsaktivität von Zellen,
**dadurch gekennzeichnet,**
daß man gegebenenfalls vorbehandelte Zellen mit mindestens einem Antikörper nach einem der Ansprüche 12 bis 23 einem Antikörperfragment nach Anspruch 24 oder einem Konjugat nach Anspruch 25 inkubiert und die Bindung des Antikörpers, des Antikörperfragments oder des Konjugats an die Zellen bestimmt.

43. Verfahren nach Anspruch 42,
**dadurch gekennzeichnet,**
daß die Vorbehandlung der Zellen, die Schritte des Fixierens oder/und Permeabilisierens umfaßt.

44. Verfahren nach Anspruch 43,
**dadurch gekennzeichnet,**
daß die Bestimmung durch eine nicht-radioaktive Markierung, insbesondere durch eine enzymatische Markierung, eine fluoreszenzchemische Markierung oder eine Lumineszenzmarkierung erfolgt.


SEQUENZPROTOKOLL

(1) ALLGEMEINE ANGABEN:

(i) ANMELDER:
(A) NAME: Boehringer Mannheim GmbH
(B) STRASSE: Sandhofer Strasse 112 - 132
(C) ORT: Mannheim
(E) LAND: Deutschland
(F) POSTLEITZAHL: 68305

(ii) BEZEICHNUNG DER ERFINDUNG: Verfahren zur Herstellung von monoklonale
Antikoerpern, die gegen proliferationsassoziierte Antigene
des Zellkerns gerichtet sind

(iii) ANZAHL DER SEQUENZEN: 7

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 12 Aminosäuren
(B) ART: Aminosäure
(C) STRANGFORM:
(D) TOPOLOGIE: linear

(xi) SEQUENZBESCHREIBUNG: SEQ ID NO: 1:

Pro Gly Xaa Asn Ala Pro Val Gly Gly Asn Val Xaa
1               5                   10

(2) ANGABEN ZU SEQ ID NO: 2:

(i) SEQUENZKENNZEICHEN:
(A) LÄNGE: 18 Aminosäuren
(B) ART: Aminosäure
(C) STRANGFORM:
(D) TOPOLOGIE: linear

(xi) SEQUENZBESCHREIBUNG: SEQ ID NO: 2:

Val Phe Arg Arg Phe Val Glu Val Gly Arg Val Ala Tyr Val Ser Phe
1               5                   10                  15

Gly Pro

(2) ANGABEN ZU SEQ ID NO: 3:

(i) SEQUENZKENNZEICHEN:
(A) LÄNGE: 224 Aminosäuren
(B) ART: Aminosäure
(C) STRANGFORM:
(D) TOPOLOGIE: linear

(xi) SEQUENZBESCHREIBUNG: SEQ ID NO: 3:

Val Phe Arg Arg Phe Val Glu Val Gly Arg Val Ala Tyr Val Ser Phe
1               5                   10                  15

Gly Pro His Ala Gly Lys Leu Val Ala Ile Val Asp Val Ile Asp Gln
            20                  25                  30

Met Arg Ala Leu Val Asp Gly Pro Cys Thr Gln Val Arg Arg Gln Ala
        35                  40                  45

Met Pro Phe Lys Cys Met Gln Leu Thr Asp Phe Ile Leu Lys Phe Pro
    50                  55                  60

His Ser Ala His Gln Lys Tyr Val Arg Gln Ala Trp Gln Lys Ala Asp
65                  70                  75                  80

Ile Asn Thr Lys Trp Ala Ala Thr Arg Trp Ala Lys Lys Ile Glu Ala
                85                  90                  95

Arg Glu Arg Lys Ala Lys Met Thr Asp Phe Xaa Arg Phe Lys Val Met
            100                 105                 110

Lys Ala Lys Lys Met Arg Asn Arg Ile Ile Lys Asn Glu Val Lys Lys
        115                 120                 125

Leu Gln Lys Ala Ala Leu Leu Lys Ala Ser Pro Lys Lys Ala Pro Gly
    130                 135                 140

Thr Lys Gly Thr Ala Ala Ala Ala Ala Ala Ala Ala Ala Ala Ala Ala
145                 150                 155                 160

Ala Lys Val Pro Ala Lys Lys Ile Thr Ala Ala Ser Lys Lys Ala Pro
                165                 170                 175

Ala Gln Lys Val Pro Ala Gln Lys Ala Thr Gly Gln Lys Ala Ala Pro
            180                 185                 190

Ala Pro Lys Ala Gln Lys Gly Gln Lys Ser Ser Ser Pro Glu Ser Thr
        195                 200                 205

Cys Ser Lys Gly Ile Trp Gln Glu Ser Ile Ser Gly Asn His Lys Lys
    210                 215                 220

(2) ANGABEN ZU SEQ ID NO: 4:

(i) SEQUENZKENNZEICHEN:
(A) LÄNGE: 1276 Basenpaare
(B) ART: Nucleotid
(C) STRANGFORM: beides
(D) TOPOLOGIE: linear

(ii) ART DES MOLEKÜLS: cDNA

(xi) SEQUENZBESCHREIBUNG: SEQ ID NO: 4:

GAATTCGGCT TTGGAGCCCG TCAGTATCGG CGGAATTCGG GTCACATTGT CACCCAGGCT 60

AGAGTGTAGT GGTGCAGTAA TGGCTCACTG CAGCCTTGAC ATCCCAGGCT CAAATGATCC 120

GTCCACCTCA GTCACTCAAG TAGCTGGGAC TACAGTCACA TGCCAGCATA CCTGACTAAT 180

TTTTGTATTT TTTCGAAGAG ATGGGGTCTC AGTATGTTGC TCAGGCTGAA CTCAAACTTC 240

TGAGCCTAAG TAATTCTCCC ACTTTAGCTC CTAAAATGTT GAAATTACAG GCATGAGCCA 300

CAGTTCATTT TTAATATTTT GGAAAACCTG CATCCTACTT TTATTGAGGG CTGCATTATT 360

CTTTTCCACC AACAGTGCAT GGAGGTTCCA AATCCTACAT ACTTGACAAC ATTGATTACT 420

TTTATTTGTT GAACAGTGGC CATGCTAATG GGTGAGAGTT AAGAGTTCAT TGAGATTTTG 480

CTTTCGATTC TTCTCATAAG TGATTTTGAT TGTCTTTTCA AATTCCTCTT GGTTATATGC 540

ATAGCTTCTT TTAGGATATG CTTCGAAGAC ACTGGTCCAC TAAAAAATGA AGTTATTCAC 600

CCTTGGTGGG TGGTTATGTT TCAGAAGTCA AATGGTGGAT GGCATTTGTA CTCCACTTAG 660

TGTTTCCCTT GATGTGTGGA TATGCAGAAG GTTTTGAAAG TTTGATATAG TACCTTTTCT 720

AATTCCCTTC TTGTTGTTCC TGCTTTTAAT GTGATATTCG AAAAACCTGC CAAAACCAAT 780

GTTACTAGGC TTCTCCCTAT ATTTTCTTCT AAGTGTTCAA GAGTTATATG TCTTTTAATT 840

TTATGTTTTT ATGTTTAGTG TGTGTAAAGT ATTTTGTTTG CATTGAAGAG AAAGGTCCAA 900

GTTCGTTATC TTTCATTTAG ACGTTGAGTT TTCTCACACC ATTGGTTGGG GAGTCTGACC 960

TTTTCTTCAC TGTTTGGTCA TCGTAACCTA GTAAAAGATT ATATGATAAT ACTTGCACAG 1020

GTTTATTTCT GGGTTCTTTG TTCTGTCATG TATCTTTTTT TCACTTTATG CCATTACCAC 1080

ATTGGTTTTA TTTTTGTAGC TTTGGACTCT TTTGACATCA TGAGGTATGG TACTTTGTTC 1140

TTTTCTAAAG CTGTTGGCTA TTCATGGTAC CTTGAGATTA CACATGAATT TTAGAATTAT 1200

ATAAAATATC TCTGCCAAAA AAAAAAAAAA AAAAAAAAAA AAAAAAAAAA AAAAAAAAAA 1260

AAAAAAAAAA AAAAAA 1276

(2) ANGABEN ZU SEQ ID NO: 5:

(i) SEQUENZKENNZEICHEN:
(A) LÄNGE: 1137 Basenpaare
(B) ART: Nucleotid
(C) STRANGFORM: beides
(D) TOPOLOGIE: linear

(ii) ART DES MOLEKÜLS: cDNA

(xi) SEQUENZBESCHREIBUNG: SEQ ID NO: 5:

GAATTCGGCT TTGGAGCCCG TCAGTATCGG CGGAATTCCA GCTGAGTGTT GGTCCTACCA 60

TTAATCNNNN AGGCGGGACT AAAACTAAAG CAAAAATCAA ACAAAATCTG GCTGAGTTTA 120

GTGGGGTGGT TGGGGAAGGT ACATAGACCC TCCTCTTGCC ACCCTAGACA GCCCTCTCAT 180

GTCTGAACCT CAGCCTGGGA GTTAGATTTA TTTGTCTCTA AATGAAGTCA GTGGATAGAT 240

GCTTGAGGAT TTTGAGTAGA AACATTCATA GTTAATTTTC ACTCTGGCCA ATCTAGTTTA 300

TGTGTGTGTT CTGGAACATT CCTCCAGCTT TTGGTGGTCA GATGGCCCAG AGATATGGGG 360

GACAGGAGGA AGAGGGTAAA TGAAGCCACA GTGAGCAGGT TCTAGGAGGT ACCTGCATCA 420

GACAAGCTGG TGGAGGCCAT GGAAGCCACA TCTACTGAGG CCTCATGCTG CTCTTGCTCT 480

GTAAGACACG GAGCCCAGGA AAGCCCATCT GCACTTCCTG AAACCTGCCT GGGGAAGATC 540

GGGGGCAGGA CCAAGTAGGC CTCATGTGTG TCTTCACCGT GCTGTCCTCA CAAGCCAGGT 600

GGGTGCCAAA GGGAGCCTGA CAGGCTGTTG TGTTAATTTA TTGTTCTTGC ACACCTGCAC 660

AGCCTCCTCT GGGGATCCCA CCTGGAGTGG ACCAGGGGTC TTGAGAAATG GAGAGTTGGC 720

TGCAAAAACT CTCATGCACT AGATGTGGCA CCTTGGAGGG CAGGGTGAGA CAAGCAGCCC 780

AGAAATACTC TCTCAAGTGG AGGGGAGAAT TTTGAGAGTG ATGGAACAGT TTGGTGGTTT 840

CAGAGAATTT CTAGGTTTCT ACTTGGATCT ACTTCTGATA CAAACTTGCA CTTGGTGCCC 900

TCTGGTGGTG TTTAGTTTTA GTTCCGTAAG AGAAATGATT CCTAGTTTGC TAAATTGGTG 960

GCATCTTTGG GAGGGGTTTC TGTTTATGGT TAGAGTCTCT TACACCCTTG TTGGAGGGAT 1020

TCTTATTCTG ACTGTGGGAG CTCCTGTTGC GGGATCTTGG GAAAAAATAA AGAAGCCGCT 1080

CATTCGCACG TCAAAACCCG AATTCCGCCG ATACTGACGG GCTCCAAAGC CGAATTC 1137

(2) ANGABEN ZU SEQ ID NO: 6:

(i) SEQUENZKENNZEICHEN:
(A) LÄNGE: 2032 Basenpaare
(B) ART: Nucleotid
(C) STRANGFORM: beides
(D) TOPOLOGIE: linear

(ii) ART DES MOLEKÜLS: cDNA

(xi) SEQUENZBESCHREIBUNG: SEQ ID NO: 6:

GAATTCGGGC CTGAAAGTGT CCAGCAGCTA CCTCAGTGAC ACCCAGAGCA GCGAGAGTCA 60

TGTTTCCAGC GTGCAGCACC CCAGGCCAGA GGAGAGCAGC CATGCCAGCC TGAGCAGCGG 120

GTACCAGGGG ACAAGGAGGG CAGCGACATC AGCTTGGTGG GCAGCCACAG AGAGTGCGGC 180

TGAACAGAAG GCTCAACACC CAGGCGGCCA GTAACCAAAC CAGCCAGCTG GCTCCATAGA 240

CCTCCAGCTC TCTAAGAGCC GGCTGACTGG CCCGCCACAG CACCAAGCAG ACTGGAGGGA 300

AAGAGTGAGG CCAGCCTGCC CGGCAGCGGC AGCGCAGCAG CATCCTGTCC AGCGCCAGCT 360
TCATCAGCTT GGGCAGCAGT GCCTCACCTA CCAGGTGAGC AGCGGGCTGC TGACTTCCAC 420
AGCCAGCCTT CCCTCCACCC AGGCCAGCAG CCGTCAGAGC GTCGGGCTGG ATGGCAGTGC 480
CCAAAACAGG CAGAGAGCCA CACCAGCGTG CAGGAGGACA AGACGGGCGG CAACGTGAAA 540
ACCAGTGAGC AGGTCACCAC GGGGGAGCAG CCTGCCCAGG TGTGCATGCC TTTGTGAGAA 600
TCCTCTCCAG CAGCCAGAGT CTCCCAGAAA ACAACACACA GGCCCAGCAA ACACAGGCTG 660
CACCCAGAGT GGATATCCCA AAACAAGTAG GAAAGGTGTT TTCAAAAGCA CACACTGACT 720
GTGTCGGCCA TCAGTGGGTA ATCCCATTTG TAGCAACAAG CATCAGTGGC AGGCCACATG 780
TAGCAGGAAG GATCTATGCT GACACCGCCT ATCCTGAGAA CACAGTCAGT GAGGAGACTG 840
TCTATACTGA GAACCACTGT TGCTAGGAAG AGCTGTAGTA TCAAGATTCA CCAATGCTGA 900
GTCCACCTGT GTCAAAACAG CGAAGAGTAA TGACATCGAC TGTGAACTTC AGTGACAAGC 960
CATCTATAAC AAGATCCATC AATGGCAAAA CCATCTGTAG TCAGCAGCAT CCTGGTGGAC 1020
CCCCTGTACC AAAACTCATC AGTCGCACAG ATGTTCTCGA TGGCAAACGC TGACGATATT 1080
AAGAACTGTC ACGAGTTAGA ACCTTCCATG ATGAAACCAT CCTAAATGAT GAATGCCATC 1140
TGCAACCAAC ACCAGCCACG ATGAGCTCAG AAGAAACAAG CTGCAACCAT GGTTGGGACT 1200
GTCCATATCA AGAACCATCT GCAGTGAGGC CCATGCGTGG AGAGCCACAT CCACATGAAA 1260
CTTCGTCCTG GCCAAGCAGC ACCACACTCT GTCCATCATT GTCACTGCCC GCAGAAGCAC 1320
TAGCAAGACA GCCACGTGCA CACGGAGGCA CCTTCACGAC TCTCAGCTCC CCCAAGGAGT 1380
CCAGACTAAG TGACTGTCAC CAGAGAAGGA TCATCAGGAG CACCAGGGAT GCCCCCGGCA 1440
CACAGGGACT AGGTCTAGTG AGAACATTAG AGGGCGGTGT AAATGGATCC CCATGCCTGG 1500
GCACCTGTAA TCCCAACTAC TTGGGAGGCA CCATCCCAGA GAGCTCCAAG CGTCCTTTTT 1560
TTTTTTTTTC CTTTGAGTGC TGTCTCTGAC ATCTTCAGCC CAGAATCTTC TCAGGAGCAA 1620
AGGCTTCCTG ATGCCTCATT CATGTCAAGA AAGGAGCCAG GAGCCATCCT GTGTTCAGAT 1680
GGTTGAGACA GTGACTACGA TGGCAGGACA ATGCCGTGAG AAGCACTTCA TGGTGACAGA 1740
GCCCAACCCA GCCGGGAACA GCTGCGATGG GGAAGCTCTG GAGCATTTTG TGAGCACCGT 1800
CTCGGTGGAT GGGAAAGCCG AAGTCTCTGC CCGTCTCTTA CTGGAGGCAC TAAACCCCCT 1860
CCCTGGGTTG ACCTCACAGA CATTGAAGTG AGTCTGCCCA CAAAGGAAAA GAGGCCAGAG 1920
ACTGTCGTGA AGACATCCAC TAGGTACCAG AAGGAAAGCT GAGGGGGCAG GAAAGACAAT 1980
AAAAGGTGTC TAGAGAGGTC AAAAAAAAAA AAAAAAAAAA AAAAAAAAAA AA 2032

(2) ANGABEN ZU SEQ ID NO: 7:

(i) SEQUENZKENNZEICHEN:
(A) LÄNGE: 971 Basenpaare
(B) ART: Nucleotid
(C) STRANGFORM: beides

(D) TOPOLOGIE: linear

(ii) ART DES MOLEKÜLS: cDNA

(xi) SEQUENZBESCHREIBUNG: SEQ ID NO: 7:

GAATTCGGCT TTGGAGCCCG TCAGTATCGG CGGAATTCGG GGGTTTGGCC AAAATGCAAG 60

TAGAAGTGGA GAAACAGAAC AGGAAAAAGT CTCCCGTCGC TGATCTTCTG CCACACATGC 120

CTCATATAAG TGAATGCTTG ATGAAAAGAA GTTTAAAACC CATCGACCTG AGAGACATGA 180

CTATTGGGCA GCTACAAGTG ATAGTCAATG ATCTCCATTC CCAGATAGAA AGCTTGAATG 240

AAGAGTTGGT CCAGCTGCTT CTCATCCGAG ATGAGCTGCA CACAGAGCAG GATGCCATGC 300

TGGTGGACAT TGAAGACTTC ACCAGACATG CTGAAGTCAG CAGAAGCACA TGGCAGAGAA 360

AATGCCTGCA AAGTGAAAAG AAGCCATTCA ACCAGAGAAC AAGCTAGAAT TTATTTTGCT 420

TCTGTGGTTG TAAAAATGCT GTTGCTAAAG GTGGCGCAGA AACAAATATC AGTGTTAGTC 480

ATTGATAATG TCTGAAGCTT AATGTCCAGT GATTGGCCTT TGCTTCTTAA TTTATTTTAA 540

TTTTTTACTT GTGCCACTTA ATATCAGGCA TTTTAATAAA ATATTGTTAC AAAAAATGTA 600

CAGTACTGAC ACCACCACAA ATCATGGTTA ATAAAAGAGA GTAGTTTTAA CTTTATTTTT 660

ATTTGTTTAG AGATTTAGTT GGAACAGTAT TTCCATTGCT ACTTTTCATT CTTCACTGTG 720

TTTTAAAGAA GAACTGTAAA TGACGGTGCT ATACAAGTCA AAAAATACAT GCCTGCCTCG 780

TAGTGAAGTT GTAGCTCTCC GTAATATGTA TATTTTACTC AGTTTTCAAC ATTTTGTGAA 840

TGTTGCTACC TGAAGTTCCT TTTTAGATGT GCTATTAACA TTCTGTTGGT TCAGAGGGTC 900

CTTGAAGTTT TATGTATAAT ATGTAACCCA GATTCCACTG AGCGCCGGTC GCTACCATTA 960

AAGCCGAATT C 971